# EUROPÄISCHE PATENTSCHRIFT

(11) **EP 3 140 398 B1**
(45) Veröffentlichungstag und Bekanntmachung des Hinweises auf die Patenterteilung: **28.08.2019**
(21) Anmeldenummer: 15744474.6
(22) Anmeldetag: 04.05.2015
(51) Int. Cl.: C12N 9/22, C12N 15/90

(54) **TAL-EFFEKTORNUKLEASE ZUM GEZIELTEN KNOCKOUT DES HIV-KOREZEPTORS CCR5**
TAL-EFFECTOR NUCLEASE FOR TARGETED KNOCKOUT OF THE HIV CO-RECEPTOR CCR5
NUCLÉASE EFFECTRICE TAL POUR LE KNOCK-OUT CIBLÉ DU CO-RÉCEPTEUR DU VIH CCR5

(30) Priorität: 07.05.2014 DE 102014106327
(43) Veröffentlichungstag der Anmeldung: 15.03.2017
(73) Patentinhaber: ADVANCED GENE & CELL TECHNOLOGIES LLC (AGCT LLC), Saint-Petersburg (RU)
(72) Erfinder: MOCK, Ulrike, London N5 1FP (GB); FEHSE, Boris, 22527 Hamburg (DE)
(74) Vertreter: Stüven, Ralf
(86) Internationale Anmeldenummer: PCT/DE2015/200295
(87) Internationale Veröffentlichungsnummer: WO 2015/169314

(56) Entgegenhaltungen:
- WO-A1-2011/146121
- WO-A1-2013/074999
- WO-A2-2012/093833
- C. MUSSOLINO ET AL: "A novel TALE nuclease scaffold enables high genome editing activity in combination with low toxicity", NUCLEIC ACIDS RESEARCH, Bd. 39, Nr. 21, 3. August 2011 (2011-08-03), Seiten 9283-9293, XP055021128, ISSN: 0305-1048, DOI: 10.1093/nar/gkr597
- SANJANA NEVILLE E ET AL: "A transcription activator-like effector toolbox for genome engineering", NATURE PROTOCOLS, NATURE PUBLISHING GROUP, GB, Bd. 7, Nr. 1, 1. Januar 2012 (2012-01-01), Seiten 171-192, XP009170390, ISSN: 1750-2799
- E. J. FINE ET AL: "An online bioinformatics tool predicts zinc finger and TALE nuclease off-target cleavage", NUCLEIC ACIDS RESEARCH, Bd. 42, Nr. 6, 30. Dezember 2013 (2013-12-30), Seiten e42-e42, XP055218748, ISSN: 0305-1048, DOI: 10.1093/nar/gkt1326

### SEQUENCE LISTING

<110> Universitätsklinikum Hamburg-Eppendorf (UKE)
<120> TAL-Effektornuklease zum gezielten Knockout des HIV-Korezeptors CCR5
<130> PAT 1473 PCT
<150> DE102014106327.9
   <151> 2014-05-07
<160> 18
<170> BiSSAP 1.3
<210> 1
   <211> 19
   <212> DNA
   <213> Homo sapiens
<400> 1
   gctggtcatc ctcatcctg 19
<210> 2
   <211> 19
   <212> DNA
   <213> Homo sapiens
<400> 2
   agatgtcagt catgctctt 19
<210> 3
   <211> 1071
   <212> PRT
   <213> Artificial Sequence
<220>
   <223> CCR5-Uco-L
<400> 3
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Gly Val Pro Ala Ala Val Asp Leu Arg Thr Leu Gly Tyr
           35                  40                  45
   Ser Gln Gln Gln Gln Glu Lys Ile Lys Pro Lys Val Arg Ser Thr Val
       50                  55                  60
   Ala Gln His His Glu Ala Leu Val Gly His Gly Phe Thr His Ala His
   65                  70                  75                  80
   Ile Val Ala Leu Ser Gln His Pro Ala Ala Leu Gly Thr Val Ala Val
                   85                  90                  95
   Lys Tyr Gln Asp Met Ile Ala Ala Leu Pro Glu Ala Thr His Glu Ala
               100                 105                 110
   Ile Val Gly Val Gly Lys Gln Trp Ser Gly Ala Arg Ala Leu Glu Ala
           115                 120                 125
   Leu Leu Thr Val Ala Gly Glu Leu Arg Gly Pro Pro Leu Gln Leu Asp
       130                 135                 140
   Thr Gly Gln Leu Leu Lys Ile Ala Lys Arg Gly Gly Val Thr Ala Val
   145                 150                 155                 160
   Glu Ala Val His Ala Trp Arg Asn Ala Leu Thr Gly Ala Pro Leu Asn
                       165                 170                 175
   Leu Thr Pro Glu Gln Val Val Ala Ile Ala Ser Asn His Gly Gly Lys
                   180                 185                 190
   Gln Ala Leu Glu Thr Val Gln Arg Leu Leu Pro Val Leu Cys Gln Ala
               195                 200                 205
   His Gly Leu Thr Pro Glu Gln Val Val Ala Ile Ala Ser His Asp Gly
       210                 215                 220
   Gly Lys Gln Ala Leu Glu Thr Val Gln Arg Leu Leu Pro Val Leu Cys
   225                 230                 235                 240
   Gln Ala His Gly Leu Thr Pro Glu Gln Val Val Ala Ile Ala Ser Asn
                   245                 250                 255
   Gly Gly Gly Lys Gln Ala Leu Glu Thr Val Gln Arg Leu Leu Pro Val
               260                 265                 270
   Leu Cys Gln Ala His Gly Leu Thr Pro Glu Gln Val Val Ala Ile Ala
           275                 280                 285
   Ser Asn His Gly Gly Lys Gln Ala Leu Glu Thr Val Gln Arg Leu Leu
       290                 295                 300
   Pro Val Leu Cys Gln Ala His Gly Leu Thr Pro Glu Gln Val Val Ala
   305                 310                 315                 320
   Ile Ala Ser Asn His Gly Gly Lys Gln Ala Leu Glu Thr Val Gln Arg
                   325                 330                 335
   Leu Leu Pro Val Leu Cys Gln Ala His Gly Leu Thr Pro Glu Gln Val
               340                 345                 350
   Val Ala Ile Ala Ser Asn Gly Gly Gly Lys Gln Ala Leu Glu Thr Val
           355                 360                 365
   Gln Arg Leu Leu Pro Val Leu Cys Gln Ala His Gly Leu Thr Pro Glu
       370                 375                 380
   Gln Val Val Ala Ile Ala Ser His Asp Gly Gly Lys Gln Ala Leu Glu
   385                 390                 395                 400
   Thr Val Gln Arg Leu Leu Pro Val Leu Cys Gln Ala His Gly Leu Thr
                   405                 410                 415
   Pro Glu Gln Val Val Ala Ile Ala Ser Asn Ile Gly Gly Lys Gln Ala
               420                 425                 430
   Leu Glu Thr Val Gln Arg Leu Leu Pro Val Leu Cys Gln Ala His Gly
           435                 440                 445
   Leu Thr Pro Glu Gln Val Val Ala Ile Val Ser Asn Gly Gly Gly Lys
       450                 455                 460
   Gln Ala Leu Glu Thr Val Gln Arg Leu Leu Pro Val Leu Cys Gln Ala
   465                 470                 475                 480
   His Gly Leu Thr Pro Glu Gln Val Val Ala Ile Ala Ser His Asp Gly
                   485                 490                 495
   Gly Lys Gln Ala Leu Glu Thr Val Gln Arg Leu Leu Pro Val Leu Cys
               500                 505                 510
   Gln Ala His Gly Leu Thr Pro Glu Gln Val Val Ala Ile Ala Ser His
           515                 520                 525
   Asp Gly Gly Lys Gln Ala Leu Glu Thr Val Gln Arg Leu Leu Pro Val
       530                 535                 540
   Leu Cys Gln Ala His Gly Leu Thr Pro Glu Gln Val Val Ala Ile Ala
   545                 550                 555                 560
   Ser Asn Gly Gly Gly Lys Gln Ala Leu Glu Thr Val Gln Arg Leu Leu
                   565                 570                 575
   Pro Val Leu Cys Gln Ala His Gly Leu Thr Pro Glu Gln Val Val Ala
               580                 585                 590
   Ile Ala Ser His Asp Gly Gly Lys Gln Ala Leu Glu Thr Val Gln Arg
           595                 600                 605
   Leu Leu Pro Val Leu Cys Gln Ala His Gly Leu Thr Pro Glu Gln Val
       610                 615                 620
   Val Ala Ile Ala Ser Asn Ile Gly Gly Lys Gln Ala Leu Glu Thr Val
   625                 630                 635                 640
   Gln Arg Leu Leu Pro Val Leu Cys Gln Ala His Gly Leu Thr Pro Glu
                   645                 650                 655
   Gln Val Val Ala Ile Ala Ser Asn Gly Gly Gly Lys Gln Ala Leu Glu
               660                 665                 670
   Thr Val Gln Arg Leu Leu Pro Val Leu Cys Gln Ala His Gly Leu Thr
           675                 680                 685
   Pro Glu Gln Val Val Ala Ile Ala Ser His Asp Gly Gly Lys Gln Ala
       690                 695                 700
   Leu Glu Thr Val Gln Arg Leu Leu Pro Val Leu Cys Gln Ala His Gly
   705                 710                 715                 720
   Leu Thr Pro Glu Gln Val Val Ala Ile Ala Ser His Asp Gly Gly Lys
                   725                 730                 735
   Gln Ala Leu Glu Thr Val Gln Arg Leu Leu Pro Val Leu Cys Gln Ala
               740                 745                 750
   His Gly Leu Thr Pro Glu Gln Val Val Ala Ile Ala Ser Asn Gly Gly
           755                 760                 765
   Gly Lys Gln Ala Leu Glu Thr Val Gln Arg Leu Leu Pro Val Leu Cys
       770                 775                 780
   Gln Ala His Gly Leu Thr Pro Glu Gln Val Val Ala Ile Ala Ser Asn
   785                 790                 795                 800
   Asn Gly Gly Arg Pro Ala Leu Glu Ser Ile Val Ala Gln Leu Ser Arg
                   805                 810                 815
   Pro Asp Pro Ala Leu Ala Ala Leu Thr Asn Asp His Leu Val Ala Leu
               820                 825                 830
   Ala Cys Leu Gly Gly Arg Pro Ala Leu Asp Ala Val Lys Lys Gly Leu
           835                 840                 845
   Pro His Ala Pro Ala Leu Ile Lys Arg Thr Asn Arg Arg Ile Pro Glu
       850                 855                 860
   Arg Thr Ser His Arg Val Ala Gly Ser Gln Leu Val Lys Ser Glu Leu
   865                 870                 875                 880
   Glu Glu Lys Lys Ser Glu Leu Arg His Lys Leu Lys Tyr Val Pro His
                   885                 890                 895
   Glu Tyr Ile Glu Leu Ile Glu Ile Ala Arg Asn Ser Thr Gln Asp Arg
               900                 905                 910
   Ile Leu Glu Met Lys Val Met Glu Phe Phe Met Lys Val Tyr Gly Tyr
           915                 920                 925
   Arg Gly Lys His Leu Gly Gly Ser Arg Lys Pro Asp Gly Ala Ile Tyr
       930                 935                 940
   Thr Val Gly Ser Pro Ile Asp Tyr Gly Val Ile Val Asp Thr Lys Ala
   945                 950                 955                 960
   Tyr Ser Gly Gly Tyr Asn Leu Pro Ile Gly Gln Ala Asp Glu Met Gln
                   965                 970                 975
   Arg Tyr Val Glu Glu Asn Gln Thr Arg Asn Lys His Ile Asn Pro Asn
               980                 985                 990
   Glu Trp Trp Lys Val Tyr Pro Ser Ser Val Thr Glu Phe Lys Phe Leu
           995                 1000                1005
   Phe Val Ser Gly His Phe Lys Gly Asn Tyr Lys Ala Gln Leu Thr Arg
       1010                1015                1020
   Leu Asn His Ile Thr Asn Cys Asn Gly Ala Val Leu Ser Val Glu Glu
   1025                1030                1035                1040
   Leu Leu Ile Gly Gly Glu Met Ile Lys Ala Gly Thr Leu Thr Leu Glu
                   1045                1050                1055
   Glu Val Arg Arg Lys Phe Asn Asn Gly Glu Ile Asn Phe Arg Ser
               1060                1065                1070
<210> 4
   <211> 1071
   <212> PRT
   <213> Artificial Sequence
<220>
   <223> CCR5-Uco-R
<400> 4
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Gly Val Pro Ala Ala Val Asp Leu Arg Thr Leu Gly Tyr
           35                  40                  45
   Ser Gln Gln Gln Gln Glu Lys Ile Lys Pro Lys Val Arg Ser Thr Val
       50                  55                  60
   Ala Gln His His Glu Ala Leu Val Gly His Gly Phe Thr His Ala His
   65                  70                  75                  80
   Ile Val Ala Leu Ser Gln His Pro Ala Ala Leu Gly Thr Val Ala Val
                   85                  90                  95
   Lys Tyr Gln Asp Met Ile Ala Ala Leu Pro Glu Ala Thr His Glu Ala
               100                 105                 110
   Ile Val Gly Val Gly Lys Gln Trp Ser Gly Ala Arg Ala Leu Glu Ala
           115                 120                 125
   Leu Leu Thr Val Ala Gly Glu Leu Arg Gly Pro Pro Leu Gln Leu Asp
       130                 135                 140
   Thr Gly Gln Leu Leu Lys Ile Ala Lys Arg Gly Gly Val Thr Ala Val
   145                 150                 155                 160
   Glu Ala Val His Ala Trp Arg Asn Ala Leu Thr Gly Ala Pro Leu Asn
                   165                 170                 175
   Leu Thr Pro Glu Gln Val Val Ala Ile Ala Ser Asn Ile Gly Gly Lys
               180                 185                 190
   Gln Ala Leu Glu Thr Val Gln Arg Leu Leu Pro Val Leu Cys Gln Ala
           195                 200                 205
   His Gly Leu Thr Pro Glu Gln Val Val Ala Ile Ala Ser Asn Asn Gly
       210                 215                 220
   Gly Lys Gln Ala Leu Glu Thr Val Gln Arg Leu Leu Pro Val Leu Cys
   225                 230                 235                 240
   Gln Ala His Gly Leu Thr Pro Glu Gln Val Val Ala Ile Ala Ser Asn
                   245                 250                 255
   Ile Gly Gly Lys Gln Ala Leu Glu Thr Val Gln Arg Leu Leu Pro Val
               260                 265                 270
   Leu Cys Gln Ala His Gly Leu Thr Pro Glu Gln Val Val Ala Ile Ala
           275                 280                 285
   Ser Asn Gly Gly Gly Lys Gln Ala Leu Glu Thr Val Gln Arg Leu Leu
       290                 295                 300
   Pro Val Leu Cys Gln Ala His Gly Leu Thr Pro Glu Gln Val Val Ala
   305                 310                 315                 320
   Ile Ala Ser Asn Asn Gly Gly Lys Gln Ala Leu Glu Thr Val Gln Arg
                   325                 330                 335
   Leu Leu Pro Val Leu Cys Gln Ala His Gly Leu Thr Pro Glu Gln Val
               340                 345                 350
   Val Ala Ile Ala Ser Asn Gly Gly Gly Lys Gln Ala Leu Glu Thr Val
           355                 360                 365
   Gln Arg Leu Leu Pro Val Leu Cys Gln Ala His Gly Leu Thr Pro Glu
       370                 375                 380
   Gln Val Val Ala Ile Ala Ser His Asp Gly Gly Lys Gln Ala Leu Glu
   385                 390                 395                 400
   Thr Val Gln Arg Leu Leu Pro Val Leu Cys Gln Ala His Gly Leu Thr
                   405                 410                 415
   Pro Glu Gln Val Val Ala Ile Ala Ser Asn Ile Gly Gly Lys Gln Ala
               420                 425                 430
   Leu Glu Thr Val Gln Arg Leu Leu Pro Val Leu Cys Gln Ala His Gly
           435                 440                 445
   Leu Thr Pro Glu Gln Val Val Ala Ile Ala Ser Asn His Gly Gly Lys
       450                 455                 460
   Gln Ala Leu Glu Thr Val Gln Arg Leu Leu Pro Val Leu Cys Gln Ala
   465                 470                 475                 480
   His Gly Leu Thr Pro Glu Gln Val Val Ala Ile Ala Ser Asn Gly Gly
                   485                 490                 495
   Gly Lys Gln Ala Leu Glu Thr Val Gln Arg Leu Leu Pro Val Leu Cys
               500                 505                 510
   Gln Ala His Gly Leu Thr Pro Glu Gln Val Val Ala Ile Ala Ser His
           515                 520                 525
   Asp Gly Gly Lys Gln Ala Leu Glu Thr Val Gln Arg Leu Leu Pro Val
       530                 535                 540
   Leu Cys Gln Ala His Gly Leu Thr Pro Glu Gln Val Val Ala Ile Ala
   545                 550                 555                 560
   Ser Asn Ile Gly Gly Lys Gln Ala Leu Glu Thr Val Gln Arg Leu Leu
                   565                 570                 575
   Pro Val Leu Cys Gln Ala His Gly Leu Thr Pro Glu Gln Val Val Ala
               580                 585                 590
   Ile Ala Ser Asn Gly Gly Gly Lys Gln Ala Leu Glu Thr Val Gln Arg
           595                 600                 605
   Leu Leu Pro Val Leu Cys Gln Ala His Gly Leu Thr Pro Glu Gln Val
       610                 615                 620
   Val Ala Ile Ala Ser Asn His Gly Gly Lys Gln Ala Leu Glu Thr Val
   625                 630                 635                 640
   Gln Arg Leu Leu Pro Val Leu Cys Gln Ala His Gly Leu Thr Pro Glu
                   645                 650                 655
   Gln Val Val Ala Ile Ala Ser His Asp Gly Gly Lys Gln Ala Leu Glu
               660                 665                 670
   Thr Val Gln Arg Leu Leu Pro Val Leu Cys Gln Ala His Gly Leu Thr
           675                 680                 685
   Pro Glu Gln Val Val Ala Ile Ala Ser Asn Gly Gly Gly Lys Gln Ala
       690                 695                 700
   Leu Glu Thr Val Gln Arg Leu Leu Pro Val Leu Cys Gln Ala His Gly
   705                 710                 715                 720
   Leu Thr Pro Glu Gln Val Val Ala Ile Ala Ser His Asp Gly Gly Lys
                   725                 730                 735
   Gln Ala Leu Glu Thr Val Gln Arg Leu Leu Pro Val Leu Cys Gln Ala
               740                 745                 750
   His Gly Leu Thr Pro Glu Gln Val Val Ala Ile Ala Ser Asn Gly Gly
           755                 760                 765
   Gly Lys Gln Ala Leu Glu Thr Val Gln Arg Leu Leu Pro Val Leu Cys
       770                 775                 780
   Gln Ala His Gly Leu Thr Pro Glu Gln Val Val Ala Ile Ala Ser Asn
   785                 790                 795                 800
   Gly Gly Gly Arg Pro Ala Leu Glu Ser Ile Val Ala Gln Leu Ser Arg
                   805                 810                 815
   Pro Asp Pro Ala Leu Ala Ala Leu Thr Asn Asp His Leu Val Ala Leu
               820                 825                 830
   Ala Cys Leu Gly Gly Arg Pro Ala Leu Asp Ala Val Lys Lys Gly Leu
           835                 840                 845
   Pro His Ala Pro Ala Leu Ile Lys Arg Thr Asn Arg Arg Ile Pro Glu
       850                 855                 860
   Arg Thr Ser His Arg Val Ala Gly Ser Gln Leu Val Lys Ser Glu Leu
   865                 870                 875                 880
   Glu Glu Lys Lys Ser Glu Leu Arg His Lys Leu Lys Tyr Val Pro His
                   885                 890                 895
   Glu Tyr Ile Glu Leu Ile Glu Ile Ala Arg Asn Ser Thr Gln Asp Arg
               900                 905                 910
   Ile Leu Glu Met Lys Val Met Glu Phe Phe Met Lys Val Tyr Gly Tyr
           915                 920                 925
   Arg Gly Lys His Leu Gly Gly Ser Arg Lys Pro Asp Gly Ala Ile Tyr
       930                 935                 940
   Thr Val Gly Ser Pro Ile Asp Tyr Gly Val Ile Val Asp Thr Lys Ala
   945                 950                 955                 960
   Tyr Ser Gly Gly Tyr Asn Leu Pro Ile Gly Gln Ala Asp Glu Met Gln
                   965                 970                 975
   Arg Tyr Val Glu Glu Asn Gln Thr Arg Asn Lys His Ile Asn Pro Asn
               980                 985                 990
   Glu Trp Trp Lys Val Tyr Pro Ser Ser Val Thr Glu Phe Lys Phe Leu
           995                 1000                1005
   Phe Val Ser Gly His Phe Lys Gly Asn Tyr Lys Ala Gln Leu Thr Arg
       1010                1015                1020
   Leu Asn His Ile Thr Asn Cys Asn Gly Ala Val Leu Ser Val Glu Glu
   1025                1030                1035                1040
   Leu Leu Ile Gly Gly Glu Met Ile Lys Ala Gly Thr Leu Thr Leu Glu
                   1045                1050                1055
   Glu Val Arg Arg Lys Phe Asn Asn Gly Glu Ile Asn Phe Arg Ser
               1060                1065                1070
<210> 5
   <211> 34
   <212> PRT
   <213> Artificial Sequence
<220>
   <223> TALEN repeat
<220>
   <221> SITE
   <222> 4
   <223> Xaa = Any amino acid, or E, Q, D or A
<220>
   <221> SITE
   <222> 10
   <223> Xaa = Any amino acid, or A or V
<220>
   <221> SITE
   <222> 12..13
   <223> Repeat variable diresidue (RVD)
<220>
   <221> SITE
   <222> 32
   <223> Xaa = Any amino acid, or A or D
<400> 5
   Leu Thr Pro Xaa Gln Val Val Ala Ile Xaa Ser Xaa Xaa Gly Gly Lys
   1               5                   10                  15
   Gln Ala Leu Glu Thr Val Gln Arg Leu Leu Pro Val Leu Cys Gln Xaa
               20                  25                  30
   His Gly
<210> 6
   <211> 34
   <212> PRT
   <213> Artificial Sequence
<220>
   <223> TALEN Repeat
<220>
   <221> SITE
   <222> 12..13
   <223> Repeat variable diresidue (RVD)
<400> 6
   Leu Thr Pro Glu Gln Val Val Ala Ile Ala Ser Xaa Xaa Gly Gly Lys
   1               5                   10                  15
   Gln Ala Leu Glu Thr Val Gln Arg Leu Leu Pro Val Leu Cys Gln Ala
               20                  25                  30
   His Gly
<210> 7
   <211> 34
   <212> PRT
   <213> Artificial Sequence
<220>
   <223> TALEN Repeat
<220>
   <221> SITE
   <222> 12..13
   <223> Repeat variable diresidue (RVD)
<400> 7
   Leu Thr Pro Gln Gln Val Val Ala Ile Ala Ser Xaa Xaa Gly Gly Lys
   1               5                   10                  15
   Gln Ala Leu Glu Thr Val Gln Arg Leu Leu Pro Val Leu Cys Gln Ala
               20                  25                  30
   His Gly
<210> 8
   <211> 34
   <212> PRT
   <213> Artificial Sequence
<220>
   <223> TALEN Repeat
<220>
   <221> SITE
   <222> 12..13
   <223> Repeat variable diresidue (RVD)
<400> 8
   Leu Thr Pro Asp Gln Val Val Ala Ile Ala Ser Xaa Xaa Gly Gly Lys
   1               5                   10                  15
   Gln Ala Leu Glu Thr Val Gln Arg Leu Leu Pro Val Leu Cys Gln Asp
               20                  25                  30
   His Gly
<210> 9
   <211> 34
   <212> PRT
   <213> Artificial Sequence
<220>
   <223> TALEN Repeat
<220>
   <221> SITE
   <222> 12..13
   <223> Repeat variable diresidue (RVD)
<400> 9
   Leu Thr Pro Ala Gln Val Val Ala Ile Ala Ser Xaa Xaa Gly Gly Lys
   1               5                   10                  15
   Gln Ala Leu Glu Thr Val Gln Arg Leu Leu Pro Val Leu Cys Gln Asp
               20                  25                  30
   His Gly
<210> 10
   <211> 34
   <212> PRT
   <213> Artificial Sequence
<220>
   <223> TALEN Repeat
<220>
   <221> SITE
   <222> 12..13
   <223> Repeat variable diresidue (RVD)
<400> 10
   Leu Thr Pro Glu Gln Val Val Ala Ile Val Ser Xaa Xaa Gly Gly Lys
   1               5                   10                  15
   Gln Ala Leu Glu Thr Val Gln Arg Leu Leu Pro Val Leu Cys Gln Ala
               20                  25                  30
   His Gly
<210> 11
   <211> 1059
   <212> DNA
   <213> Homo sapiens
<220>
   <223> hCCR5 DNA
<400> 11
   atggattatc aagtgtcaag tccaatctat gacatcaatt attatacatc ggagccctgc      60
   caaaaaatca atgtgaagca aatcgcagcc cgcctcctgc ctccgctcta ctcactggtg     120
   ttcatctttg gttttgtggg caacatgctg gtcatcctca tcctgataaa ctgcaaaagg     180
   ctgaagagca tgactgacat ctacctgctc aacctggcca tctctgacct gtttttcctt     240
   cttactgtcc ccttctgggc tcactatgct gccgcccagt gggactttgg aaatacaatg     300
   tgtcaactct tgacagggct ctattttata ggcttcttct ctggaatctt cttcatcatc     360
   ctcctgacaa tcgataggta cctggctgtc gtccatgctg tgtttgcttt aaaagccagg     420
   acggtcacct ttggggtggt gacaagtgtg atcacttggg tggtggctgt gtttgcgtct     480
   ctcccaggaa tcatctttac cagatctcaa aaagaaggtc ttcattacac ctgcagctct     540
   cattttccat acagtcagta tcaattctgg aagaatttcc agacattaaa gatagtcatc     600
   ttggggctgg tcctgccgct gcttgtcatg gtcatctgct actcgggaat cctaaaaact     660
   ctgcttcggt gtcgaaatga gaagaagagg cacagggctg tgaggcttat cttcaccatc     720
   atgattgttt attttctctt ctgggctccc tacaacattg tccttctcct gaacaccttc     780
   caggaattct ttggcctgaa taattgcagt agctctaaca ggttggacca agctatgcag     840
   gtgacagaga ctcttgggat gacgcactgc tgcatcaacc ccatcatcta tgcctttgtc     900
   ggggagaagt tcagaaacta cctcttagtc ttcttccaaa agcacattgc caaacgcttc     960
   tgcaaatgct gttctatttt ccagcaagag gctcccgagc gagcaagctc agtttacacc    1020
   cgatccactg gggagcagga aatatctgtg ggcttgtga                           1059
<210> 12
   <211> 198
   <212> PRT
   <213> Planomicrobium okeanokoites
<220>
   <223> FokI cleavage domain
<400> 12
   Gln Leu Val Lys Ser Glu Leu Glu Glu Lys Lys Ser Glu Leu Arg His
   1               5                   10                  15
   Lys Leu Lys Tyr Val Pro His Glu Tyr Ile Glu Leu Ile Glu Ile Ala
               20                  25                  30
   Arg Asn Ser Thr Gln Asp Arg Ile Leu Glu Met Lys Val Met Glu Phe
           35                  40                  45
   Phe Met Lys Val Tyr Gly Tyr Arg Gly Lys His Leu Gly Gly Ser Arg
       50                  55                  60
   Lys Pro Asp Gly Ala Ile Tyr Thr Val Gly Ser Pro Ile Asp Tyr Gly
   65                  70                  75                  80
   Val Ile Val Asp Thr Lys Ala Tyr Ser Gly Gly Tyr Asn Leu Pro Ile
                   85                  90                  95
   Gly Gln Ala Asp Glu Met Gln Arg Tyr Val Glu Glu Asn Gln Thr Arg
               100                 105                 110
   Asn Lys His Ile Asn Pro Asn Glu Trp Trp Lys Val Tyr Pro Ser Ser
           115                 120                 125
   Val Thr Glu Phe Lys Phe Leu Phe Val Ser Gly His Phe Lys Gly Asn
       130                 135                 140
   Tyr Lys Ala Gln Leu Thr Arg Leu Asn His Ile Thr Asn Cys Asn Gly
   145                 150                 155                 160
   Ala Val Leu Ser Val Glu Glu Leu Leu Ile Gly Gly Glu Met Ile Lys
                   165                 170                 175
   Ala Gly Thr Leu Thr Leu Glu Glu Val Arg Arg Lys Phe Asn Asn Gly
               180                 185                 190
   Glu Ile Asn Phe Arg Ser
           195
<210> 13
   <211> 1037
   <212> PRT
   <213> Artificial Sequence
<220>
   <223> CCR5-Mco-L
<400> 13
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Gly Val Pro Ala Ala Val Asp Leu Arg Thr Leu Gly Tyr
           35                  40                  45
   Ser Gln Gln Gln Gln Glu Lys Ile Lys Pro Lys Val Arg Ser Thr Val
       50                  55                  60
   Ala Gln His His Glu Ala Leu Val Gly His Gly Phe Thr His Ala His
   65                  70                  75                  80
   Ile Val Ala Leu Ser Gln His Pro Ala Ala Leu Gly Thr Val Ala Val
                   85                  90                  95
   Lys Tyr Gln Asp Met Ile Ala Ala Leu Pro Glu Ala Thr His Glu Ala
               100                 105                 110
   Ile Val Gly Val Gly Lys Gln Trp Ser Gly Ala Arg Ala Leu Glu Ala
           115                 120                 125
   Leu Leu Thr Val Ala Gly Glu Leu Arg Gly Pro Pro Leu Gln Leu Asp
       130                 135                 140
   Thr Gly Gln Leu Leu Lys Ile Ala Lys Arg Gly Gly Val Thr Ala Val
   145                 150                 155                 160
   Glu Ala Val His Ala Trp Arg Asn Ala Leu Thr Gly Ala Pro Leu Asn
                   165                 170                 175
   Leu Thr Pro Glu Gln Val Val Ala Ile Ala Ser Asn Asn Gly Gly Lys
               180                 185                 190
   Gln Ala Leu Glu Thr Val Gln Arg Leu Leu Pro Val Leu Cys Gln Ala
           195                 200                 205
   His Gly Leu Thr Pro Glu Gln Val Val Ala Ile Ala Ser Asn Gly Gly
       210                 215                 220
   Gly Lys Gln Ala Leu Glu Thr Val Gln Arg Leu Leu Pro Val Leu Cys
   225                 230                 235                 240
   Gln Ala His Gly Leu Thr Pro Glu Gln Val Val Ala Ile Ala Ser Asn
                   245                 250                 255
   Asn Gly Gly Lys Gln Ala Leu Glu Thr Val Gln Arg Leu Leu Pro Val
               260                 265                 270
   Leu Cys Gln Ala His Gly Leu Thr Pro Glu Gln Val Val Ala Ile Ala
           275                 280                 285
   Ser Asn Asn Gly Gly Lys Gln Ala Leu Glu Thr Val Gln Arg Leu Leu
       290                 295                 300
   Pro Val Leu Cys Gln Ala His Gly Leu Thr Pro Glu Gln Val Val Ala
   305                 310                 315                 320
   Ile Ala Ser Asn Asn Gly Gly Lys Gln Ala Leu Glu Thr Val Gln Arg
                   325                 330                 335
   Leu Leu Pro Val Leu Cys Gln Ala His Gly Leu Thr Pro Glu Gln Val
               340                 345                 350
   Val Ala Ile Ala Ser His Asp Gly Gly Lys Gln Ala Leu Glu Thr Val
           355                 360                 365
   Gln Arg Leu Leu Pro Val Leu Cys Gln Ala His Gly Leu Thr Pro Glu
       370                 375                 380
   Gln Val Val Ala Ile Ala Ser Asn Ile Gly Gly Lys Gln Ala Leu Glu
   385                 390                 395                 400
   Thr Val Gln Arg Leu Leu Pro Val Leu Cys Gln Ala His Gly Leu Thr
                   405                 410                 415
   Pro Glu Gln Val Val Ala Ile Ala Ser Asn Ile Gly Gly Lys Gln Ala
               420                 425                 430
   Leu Glu Thr Val Gln Arg Leu Leu Pro Val Leu Cys Gln Ala His Gly
           435                 440                 445
   Leu Thr Pro Glu Gln Val Val Ala Ile Ala Ser His Asp Gly Gly Lys
       450                 455                 460
   Gln Ala Leu Glu Thr Val Gln Arg Leu Leu Pro Val Leu Cys Gln Ala
   465                 470                 475                 480
   His Gly Leu Thr Pro Glu Gln Val Val Ala Ile Ala Ser Asn Ile Gly
                   485                 490                 495
   Gly Lys Gln Ala Leu Glu Thr Val Gln Arg Leu Leu Pro Val Leu Cys
               500                 505                 510
   Gln Ala His Gly Leu Thr Pro Glu Gln Val Val Ala Ile Ala Ser Asn
           515                 520                 525
   Gly Gly Gly Lys Gln Ala Leu Glu Thr Val Gln Arg Leu Leu Pro Val
       530                 535                 540
   Leu Cys Gln Ala His Gly Leu Thr Pro Glu Gln Val Val Ala Ile Ala
   545                 550                 555                 560
   Ser Asn Asn Gly Gly Lys Gln Ala Leu Glu Thr Val Gln Arg Leu Leu
                   565                 570                 575
   Pro Val Leu Cys Gln Ala His Gly Leu Thr Pro Glu Gln Val Val Ala
               580                 585                 590
   Ile Ala Ser His Asp Gly Gly Lys Gln Ala Leu Glu Thr Val Gln Arg
           595                 600                 605
   Leu Leu Pro Val Leu Cys Gln Ala His Gly Leu Thr Pro Glu Gln Val
       610                 615                 620
   Val Ala Ile Ala Ser Asn Gly Gly Gly Lys Gln Ala Leu Glu Thr Val
   625                 630                 635                 640
   Gln Arg Leu Leu Pro Val Leu Cys Gln Ala His Gly Leu Thr Pro Glu
                   645                 650                 655
   Gln Val Val Ala Ile Ala Ser Asn Asn Gly Gly Lys Gln Ala Leu Glu
               660                 665                 670
   Thr Val Gln Arg Leu Leu Pro Val Leu Cys Gln Ala His Gly Leu Thr
           675                 680                 685
   Pro Glu Gln Val Val Ala Ile Ala Ser Asn Asn Gly Gly Lys Gln Ala
       690                 695                 700
   Leu Glu Thr Val Gln Arg Leu Leu Pro Val Leu Cys Gln Ala His Gly
   705                 710                 715                 720
   Leu Thr Pro Glu Gln Val Val Ala Ile Ala Ser Asn Gly Gly Gly Lys
                   725                 730                 735
   Gln Ala Leu Glu Thr Val Gln Arg Leu Leu Pro Val Leu Cys Gln Ala
               740                 745                 750
   His Gly Leu Thr Pro Glu Gln Val Val Ala Ile Ala Ser His Asp Gly
           755                 760                 765
   Gly Arg Pro Ala Leu Glu Ser Ile Val Ala Gln Leu Ser Arg Pro Asp
       770                 775                 780
   Pro Ala Leu Ala Ala Leu Thr Asn Asp His Leu Val Ala Leu Ala Cys
   785                 790                 795                 800
   Leu Gly Gly Arg Pro Ala Leu Asp Ala Val Lys Lys Gly Leu Pro His
                   805                 810                 815
   Ala Pro Ala Leu Ile Lys Arg Thr Asn Arg Arg Ile Pro Glu Arg Thr
               820                 825                 830
   Ser His Arg Val Ala Gly Ser Gln Leu Val Lys Ser Glu Leu Glu Glu
           835                 840                 845
   Lys Lys Ser Glu Leu Arg His Lys Leu Lys Tyr Val Pro His Glu Tyr
       850                 855                 860
   Ile Glu Leu Ile Glu Ile Ala Arg Asn Ser Thr Gln Asp Arg Ile Leu
   865                 870                 875                 880
   Glu Met Lys Val Met Glu Phe Phe Met Lys Val Tyr Gly Tyr Arg Gly
                   885                 890                 895
   Lys His Leu Gly Gly Ser Arg Lys Pro Asp Gly Ala Ile Tyr Thr Val
               900                 905                 910
   Gly Ser Pro Ile Asp Tyr Gly Val Ile Val Asp Thr Lys Ala Tyr Ser
           915                 920                 925
   Gly Gly Tyr Asn Leu Pro Ile Gly Gln Ala Asp Glu Met Gln Arg Tyr
       930                 935                 940
   Val Glu Glu Asn Gln Thr Arg Asn Lys His Ile Asn Pro Asn Glu Trp
   945                 950                 955                 960
   Trp Lys Val Tyr Pro Ser Ser Val Thr Glu Phe Lys Phe Leu Phe Val
                   965                 970                 975
   Ser Gly His Phe Lys Gly Asn Tyr Lys Ala Gln Leu Thr Arg Leu Asn
               980                 985                 990
   His Ile Thr Asn Cys Asn Gly Ala Val Leu Ser Val Glu Glu Leu Leu
           995                 1000                1005
   Ile Gly Gly Glu Met Ile Lys Ala Gly Thr Leu Thr Leu Glu Glu Val
       1010                1015                1020
   Arg Arg Lys Phe Asn Asn Gly Glu Ile Asn Phe Arg Ser
   1025                1030                1035
<210> 14
   <211> 1037
   <212> PRT
   <213> Artificial Sequence
<220>
   <223> CCR5-Mco-R
<400> 14
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Gly Val Pro Ala Ala Val Asp Leu Arg Thr Leu Gly Tyr
           35                  40                  45
   Ser Gln Gln Gln Gln Glu Lys Ile Lys Pro Lys Val Arg Ser Thr Val
       50                  55                  60
   Ala Gln His His Glu Ala Leu Val Gly His Gly Phe Thr His Ala His
   65                  70                  75                  80
   Ile Val Ala Leu Ser Gln His Pro Ala Ala Leu Gly Thr Val Ala Val
                   85                  90                  95
   Lys Tyr Gln Asp Met Ile Ala Ala Leu Pro Glu Ala Thr His Glu Ala
               100                 105                 110
   Ile Val Gly Val Gly Lys Gln Trp Ser Gly Ala Arg Ala Leu Glu Ala
           115                 120                 125
   Leu Leu Thr Val Ala Gly Glu Leu Arg Gly Pro Pro Leu Gln Leu Asp
       130                 135                 140
   Thr Gly Gln Leu Leu Lys Ile Ala Lys Arg Gly Gly Val Thr Ala Val
   145                 150                 155                 160
   Glu Ala Val His Ala Trp Arg Asn Ala Leu Thr Gly Ala Pro Leu Asn
                   165                 170                 175
   Leu Thr Pro Glu Gln Val Val Ala Ile Ala Ser His Asp Gly Gly Lys
               180                 185                 190
   Gln Ala Leu Glu Thr Val Gln Arg Leu Leu Pro Val Leu Cys Gln Ala
           195                 200                 205
   His Gly Leu Thr Pro Glu Gln Val Val Ala Ile Ala Ser Asn Gly Gly
       210                 215                 220
   Gly Lys Gln Ala Leu Glu Thr Val Gln Arg Leu Leu Pro Val Leu Cys
   225                 230                 235                 240
   Gln Ala His Gly Leu Thr Pro Glu Gln Val Val Ala Ile Ala Ser Asn
                   245                 250                 255
   Gly Gly Gly Lys Gln Ala Leu Glu Thr Val Gln Arg Leu Leu Pro Val
               260                 265                 270
   Leu Cys Gln Ala His Gly Leu Thr Pro Glu Gln Val Val Ala Ile Ala
           275                 280                 285
   Ser His Asp Gly Gly Lys Gln Ala Leu Glu Thr Val Gln Arg Leu Leu
       290                 295                 300
   Pro Val Leu Cys Gln Ala His Gly Leu Thr Pro Glu Gln Val Val Ala
   305                 310                 315                 320
   Ile Ala Ser Asn Ile Gly Gly Lys Gln Ala Leu Glu Thr Val Gln Arg
                   325                 330                 335
   Leu Leu Pro Val Leu Cys Gln Ala His Gly Leu Thr Pro Glu Gln Val
               340                 345                 350
   Val Ala Ile Ala Ser Asn Asn Gly Gly Lys Gln Ala Leu Glu Thr Val
           355                 360                 365
   Gln Arg Leu Leu Pro Val Leu Cys Gln Ala His Gly Leu Thr Pro Glu
       370                 375                 380
   Gln Val Val Ala Ile Ala Ser His Asp Gly Gly Lys Gln Ala Leu Glu
   385                 390                 395                 400
   Thr Val Gln Arg Leu Leu Pro Val Leu Cys Gln Ala His Gly Leu Thr
                   405                 410                 415
   Pro Glu Gln Val Val Ala Ile Ala Ser His Asp Gly Gly Lys Gln Ala
               420                 425                 430
   Leu Glu Thr Val Gln Arg Leu Leu Pro Val Leu Cys Gln Ala His Gly
           435                 440                 445
   Leu Thr Pro Glu Gln Val Val Ala Ile Ala Ser Asn Gly Gly Gly Lys
       450                 455                 460
   Gln Ala Leu Glu Thr Val Gln Arg Leu Leu Pro Val Leu Cys Gln Ala
   465                 470                 475                 480
   His Gly Leu Thr Pro Glu Gln Val Val Ala Ile Ala Ser Asn Gly Gly
                   485                 490                 495
   Gly Lys Gln Ala Leu Glu Thr Val Gln Arg Leu Leu Pro Val Leu Cys
                       500                 505                 510
   Gln Ala His Gly Leu Thr Pro Glu Gln Val Val Ala Ile Ala Ser Asn
               515                 520                 525
   Gly Gly Gly Lys Gln Ala Leu Glu Thr Val Gln Arg Leu Leu Pro Val
       530                 535                 540
   Leu Cys Gln Ala His Gly Leu Thr Pro Glu Gln Val Val Ala Ile Ala
   545                 550                 555                 560
   Ser Asn Gly Gly Gly Lys Gln Ala Leu Glu Thr Val Gln Arg Leu Leu
                   565                 570                 575
   Pro Val Leu Cys Gln Ala His Gly Leu Thr Pro Glu Gln Val Val Ala
               580                 585                 590
   Ile Ala Ser Asn Asn Gly Gly Lys Gln Ala Leu Glu Thr Val Gln Arg
           595                 600                 605
   Leu Leu Pro Val Leu Cys Gln Ala His Gly Leu Thr Pro Glu Gln Val
       610                 615                 620
   Val Ala Ile Ala Ser His Asp Gly Gly Lys Gln Ala Leu Glu Thr Val
   625                 630                 635                 640
   Gln Arg Leu Leu Pro Val Leu Cys Gln Ala His Gly Leu Thr Pro Glu
                   645                 650                 655
   Gln Val Val Ala Ile Ala Ser Asn Ile Gly Gly Lys Gln Ala Leu Glu
               660                 665                 670
   Thr Val Gln Arg Leu Leu Pro Val Leu Cys Gln Ala His Gly Leu Thr
           675                 680                 685
   Pro Glu Gln Val Val Ala Ile Ala Ser Asn Asn Gly Gly Lys Gln Ala
       690                 695                 700
   Leu Glu Thr Val Gln Arg Leu Leu Pro Val Leu Cys Gln Ala His Gly
   705                 710                 715                 720
   Leu Thr Pro Glu Gln Val Val Ala Ile Ala Ser Asn Gly Gly Gly Lys
                   725                 730                 735
   Gln Ala Leu Glu Thr Val Gln Arg Leu Leu Pro Val Leu Cys Gln Ala
               740                 745                 750
   His Gly Leu Thr Pro Glu Gln Val Val Ala Ile Ala Ser Asn Gly Gly
           755                 760                 765
   Gly Arg Pro Ala Leu Glu Ser Ile Val Ala Gln Leu Ser Arg Pro Asp
       770                 775                 780
   Pro Ala Leu Ala Ala Leu Thr Asn Asp His Leu Val Ala Leu Ala Cys
   785                 790                 795                 800
   Leu Gly Gly Arg Pro Ala Leu Asp Ala Val Lys Lys Gly Leu Pro His
                   805                 810                 815
   Ala Pro Ala Leu Ile Lys Arg Thr Asn Arg Arg Ile Pro Glu Arg Thr
               820                 825                 830
   Ser His Arg Val Ala Gly Ser Gln Leu Val Lys Ser Glu Leu Glu Glu
           835                 840                 845
   Lys Lys Ser Glu Leu Arg His Lys Leu Lys Tyr Val Pro His Glu Tyr
       850                 855                 860
   Ile Glu Leu Ile Glu Ile Ala Arg Asn Ser Thr Gln Asp Arg Ile Leu
   865                 870                 875                 880
   Glu Met Lys Val Met Glu Phe Phe Met Lys Val Tyr Gly Tyr Arg Gly
                   885                 890                 895
   Lys His Leu Gly Gly Ser Arg Lys Pro Asp Gly Ala Ile Tyr Thr Val
               900                 905                 910
   Gly Ser Pro Ile Asp Tyr Gly Val Ile Val Asp Thr Lys Ala Tyr Ser
           915                 920                 925
   Gly Gly Tyr Asn Leu Pro Ile Gly Gln Ala Asp Glu Met Gln Arg Tyr
       930                 935                 940
   Val Glu Glu Asn Gln Thr Arg Asn Lys His Ile Asn Pro Asn Glu Trp
   945                 950                 955                 960
   Trp Lys Val Tyr Pro Ser Ser Val Thr Glu Phe Lys Phe Leu Phe Val
                   965                 970                 975
   Ser Gly His Phe Lys Gly Asn Tyr Lys Ala Gln Leu Thr Arg Leu Asn
               980                 985                 990
   His Ile Thr Asn Cys Asn Gly Ala Val Leu Ser Val Glu Glu Leu Leu
           995                 1000                1005
   Ile Gly Gly Glu Met Ile Lys Ala Gly Thr Leu Thr Leu Glu Glu Val
       1010                1015                1020
   Arg Arg Lys Phe Asn Asn Gly Glu Ile Asn Phe Arg Ser
   1025                1030                1035
<210> 15
   <211> 18
   <212> DNA
   <213> Homo sapiens
<400> 15
   gtgggcaaca tgctggtc 18
<210> 16
   <211> 18
   <212> PRT
   <213> Homo sapiens
<400> 16
   Cys Thr Thr Cys Ala Gly Cys Cys Thr Thr Thr Thr Gly Cys Ala Gly
   1               5                   10                  15
   Thr Thr
<210> 17
   <211> 3788
   <212> RNA
   <213> Artificial Sequence
<220>
   <223> CCR5-Uco-L mRNA
<400> 17
   ggggccacca uggacuauaa ggaccacgac ggagacuaca aggaucauga uauugauuac      60
   aaagacgaug acgauaagau ggccccaaag aagaagcgga aggucgguau ccacggaguc     120
   ccagcagccg uagauuugag aacuuuggga uauucacagc agcagcagga aaagaucaag     180
   cccaaaguga ggucgacagu cgcgcagcau cacgaagcgc ugguggguca uggguuuaca     240
   caugcccaca ucguagccuu gucgcagcac ccugcagccc uuggcacggu cgccgucaag     300
   uaccaggaca ugauugcggc guugccggaa gccacacaug aggcgaucgu cggugugggg     360
   aaacagugga gcggagcccg agcgcuugag gcccuguuga cggucgcggg agagcugaga     420
   gggccucccc uucagcugga cacgggccag uugcugaaga ucgcgaagcg gggaggaguc     480
   acggcggucg aggcggugca cgcguggcgc aaugcgcuca cgggagcacc ccucaaccug     540
   accccagagc aggucguggc aauugcgagc aaccacgggg gaaagcaggc acucgaaacc     600
   guccagaggu ugcugccugu gcugugccaa gcgcacggac uuacgccaga gcaggucgug     660
   gcaauugcga gccaugacgg gggaaagcag gcacucgaaa ccguccagag guugcugccu     720
   gugcugugcc aagcgcacgg acuaacccca gagcaggucg uggcaauugc gagcaacgga     780
   gggggaaagc aggcacucga aaccguccag agguugcugc cugugcugug ccaagcgcac     840
   ggguugaccc cagagcaggu cguggcaauu gcgagcaacc acgggggaaa gcaggcacuc     900
   gaaaccgucc agagguugcu gccugugcug ugccaagcgc acggccugac cccagagcag 960
   gucguggcaa uugcgagcaa ccacggggga aagcaggcac ucgaaaccgu ccagagguug 1020
   cugccugugc ugugccaagc gcacggacug acaccagagc aggucguggc aauugcgagc 1080
   aacggagggg gaaagcaggc acucgaaacc guccagaggu ugcugccugu gcugugccaa 1140
   gcgcacggac uuacacccga acaagucgug gcaauugcga gccaugacgg gggaaagcag 1200
   gcacucgaaa ccguccagag guugcugccu gugcugugcc aagcgcacgg acuuacgcca 1260
   gagcaggucg uggcaauugc gagcaacauc gggggaaagc aggcacucga aaccguccag 1320
   agguugcugc cugugcugug ccaagcgcac ggacuaaccc cagagcaggu cguggcaauu 1380
   gugagcaacg gagggggaaa gcaggcacuc gaaaccgucc agagguugcu gccugugcug 1440
   ugccaagcgc acggguugac cccagagcag gucguggcaa uugcgagcca ugacggggga 1500
   aagcaggcac ucgaaaccgu ccagagguug cugccugugc ugugccaagc gcacggccug 1560
   accccagagc aggucguggc aauugcgagc caugacgggg gaaagcaggc acucgaaacc 1620
   guccagaggu ugcugccugu gcugugccaa gcgcacggac ugacaccaga gcaggucgug 1680
   gcaauugcga gcaacggagg gggaaagcag gcacucgaaa ccguccagag guugcugccu 1740
   gugcugugcc aagcgcacgg ccucacccca gagcaggucg uggcaauugc gagccaugac 1800
   gggggaaagc aggcacucga aaccguccag agguugcugc cugugcugug ccaagcgcac 1860
   ggacuuacgc cagagcaggu cguggcaauu gcgagcaaca ucgggggaaa gcaggcacuc 1920
   gaaaccgucc agagguugcu gccugugcug ugccaagcgc acggacuaac cccagagcag 1980
   gucguggcaa uugcgagcaa cggaggggga aagcaggcac ucgaaaccgu ccagagguug 2040
   cugccugugc ugugccaagc gcacggguug accccagagc aggucguggc aauugcgagc 2100
   caugacgggg gaaagcaggc acucgaaacc guccagaggu ugcugccugu gcugugccaa 2160
   gcgcacggcc ugaccccaga gcaggucgug gcaauugcga gccaugacgg gggaaagcag 2220
   gcacucgaaa ccguccagag guugcugccu gugcugugcc aagcgcacgg acugacacca 2280
   gagcaggucg uggcaauugc gagcaacgga gggggaaagc aggcacucga aaccguccag 2340
   agguugcugc cugugcugug ccaagcgcac ggacucacgc cugagcaggu aguggcuauu 2400
   gcauccaaca acgggggcag acccgcacug gagucaaucg uggcccagcu uucgaggccg 2460
   gaccccgcgc uggccgcacu cacuaaugau caucuuguag cgcuggccug ccucggcgga 2520
   cgacccgccu uggaugcggu gaagaagggg cucccgcacg cgccugcauu gauuaagcgg 2580
   accaacagaa ggauucccga gaggacauca caucgagugg cagguuccca acucgugaag 2640
   agugaacuug aggagaaaaa gucggagcug cggcacaaau ugaaauacgu accgcaugaa 2700
   uacaucgaac uuaucgaaau ugcuaggaac ucgacucaag acagaauccu ugagaugaag 2760
   guaauggagu ucuuuaugaa gguuuaugga uaccgaggga agcaucucgg uggaucacga 2820
   aaacccgacg gagcaaucua uacggugggg agcccgauug auuacggagu gaucgucgac 2880
   acgaaagccu acagcggugg guacaaucuu cccaucgggc aggcagauga gaugcaacgu 2940
   uaugucgaag aaaaucagac caggaacaaa cacaucaauc caaaugagug guggaaagug 3000
   uauccuucau cagugaccga guuuaaguuu uuguuugucu cugggcauuu caaaggcaac 3060
   uauaaggccc agcucacacg guugaaucac auuacgaacu gcaauggugc gguuuugucc 3120
   guagaggaac ugcucauugg uggagaaaug aucaaagcgg gaacucugac acuggaagaa 3180
   gucagacgca aguuuaacaa uggcgagauc aauuuccgcu cauaaaaaau cagccucgac 3240
   ugugccuucu aguugccagc caucuguugu uugccccucc cccgugccuu ccuugacccu 3300
   ggaaggugcc acucccacug uccuuuccua auaaaaugag gaaauugcau cacaacacuc 3360
   aacccuaucu cggucuauuc uuuugauuua uaagggauuu ugccgauuuc ggccuauugg 3420
   uuaaaaaaug agcugauuua acaaaaauuu aacgcgaauu aauucugugg aauguguguc 3480
   aguuagggug uggaaagucc ccaggcuccc cagcaggcag aaguaugcaa agcaugcauc 3540
   ucaauuaguc agcaaccagg uguggaaagu ccccaggcuc cccagcaggc agaaguaugc 3600
   aaagcaugca ucucaauuag ucagcaacca uagucccgcc ccuaacuccg cccaucccgc 3660
   cccuaacucc gcccaguucc gcccauucuc cgccccaugg cugacuaauu uuuuuuauuu 3720
   augcagaggc cgaggccgcc ucugccucug agcuauucca gaaguaguga ggaggcuuuu 3780
   uuggaggc 3788
<210> 18
   <211> 3788
   <212> RNA
   <213> Artificial Sequence
<220>
   <223> CCR5-Uco-R mRNA
<400> 18
   ggggccacca uggacuauaa ggaccacgac ggagacuaca aggaucauga uauugauuac        60
   aaagacgaug acgauaagau ggccccaaag aagaagcgga aggucgguau ccacggaguc       120
   ccagcagccg uagauuugag aacuuuggga uauucacagc agcagcagga aaagaucaag       180
   cccaaaguga ggucgacagu cgcgcagcau cacgaagcgc ugguggguca uggguuuaca       240
   caugcccaca ucguagccuu gucgcagcac ccugcagccc uuggcacggu cgccgucaag       300
   uaccaggaca ugauugcggc guugccggaa gccacacaug aggcgaucgu cggugugggg       360
   aaacagugga gcggagcccg agcgcuugag gcccuguuga cggucgcggg agagcugaga       420
   gggccucccc uucagcugga cacgggccag uugcugaaga ucgcgaagcg gggaggaguc       480
   acggcggucg aggcggugca cgcguggcgc aaugcgcuca cgggagcacc ccucaaccug       540
   accccagagc aggucguggc aauugcgagc aacaucgggg gaaagcaggc acucgaaacc 600
   guccagaggu ugcugccugu gcugugccaa gcgcacggac uuacgccaga gcaggucgug 660
   gcaauugcga gcaacaacgg gggaaagcag gcacucgaaa ccguccagag guugcugccu 720
   gugcugugcc aagcgcacgg acuaacccca gagcaggucg uggcaauugc gagcaacauc 780
   gggggaaagc aggcacucga aaccguccag agguugcugc cugugcugug ccaagcgcac 840
   ggguugaccc cagagcaggu cguggcaauu gcgagcaacg gagggggaaa gcaggcacuc 900
   gaaaccgucc agagguugcu gccugugcug ugccaagcgc acggccugac cccagagcag 960
   gucguggcaa uugcgagcaa caacggggga aagcaggcac ucgaaaccgu ccagagguug 1020
   cugccugugc ugugccaagc gcacggacug acaccagagc aggucguggc aauugcgagc 1080
   aacggagggg gaaagcaggc acucgaaacc guccagaggu ugcugccugu gcugugccaa 1140
   gcgcacggac uuacacccga acaagucgug gcaauugcga gccaugacgg gggaaagcag 1200
   gcacucgaaa ccguccagag guugcugccu gugcugugcc aagcgcacgg acuuacgcca 1260
   gagcaggucg uggcaauugc gagcaacauc gggggaaagc aggcacucga aaccguccag 1320
   agguugcugc cugugcugug ccaagcgcac ggacuaaccc cagagcaggu cguggcaauu 1380
   gcgagcaacc acgggggaaa gcaggcacuc gaaaccgucc agagguugcu gccugugcug 1440
   ugccaagcgc acggguugac cccagagcag gucguggcaa uugcgagcaa cggaggggga 1500
   aagcaggcac ucgaaaccgu ccagagguug cugccugugc ugugccaagc gcacggccug 1560
   accccagagc aggucguggc aauugcgagc caugacgggg gaaagcaggc acucgaaacc 1620
   guccagaggu ugcugccugu gcugugccaa gcgcacggac ugacaccaga gcaggucgug 1680
   gcaauugcga gcaacaucgg gggaaagcag gcacucgaaa ccguccagag guugcugccu 1740
   gugcugugcc aagcgcacgg ccucacccca gagcaggucg uggcaauugc gagcaacgga 1800
   gggggaaagc aggcacucga aaccguccag agguugcugc cugugcugug ccaagcgcac 1860
   ggacuuacgc cagagcaggu cguggcaauu gcgagcaacc acgggggaaa gcaggcacuc 1920
   gaaaccgucc agagguugcu gccugugcug ugccaagcgc acggacuaac cccagagcag 1980
   gucguggcaa uugcgagcca ugacggggga aagcaggcac ucgaaaccgu ccagagguug 2040
   cugccugugc ugugccaagc gcacggguug accccagagc aggucguggc aauugcgagc 2100
   aacggagggg gaaagcaggc acucgaaacc guccagaggu ugcugccugu gcugugccaa 2160
   gcgcacggcc ugaccccaga gcaggucgug gcaauugcga gccaugacgg gggaaagcag 2220
   gcacucgaaa ccguccagag guugcugccu gugcugugcc aagcgcacgg acugacacca 2280
   gagcaggucg uggcaauugc gagcaacgga gggggaaagc aggcacucga aaccguccag 2340
   agguugcugc cugugcugug ccaagcgcac ggacucacgc cugagcaggu aguggcuauu 2400
   gcauccaacg gagggggcag acccgcacug gagucaaucg uggcccagcu uucgaggccg 2460
   gaccccgcgc uggccgcacu cacuaaugau caucuuguag cgcuggccug ccucggcgga 2520
   cgacccgccu uggaugcggu gaagaagggg cucccgcacg cgccugcauu gauuaagcgg 2580
   accaacagaa ggauucccga gaggacauca caucgagugg cagguuccca acucgugaag 2640
   agugaacuug aggagaaaaa gucggagcug cggcacaaau ugaaauacgu accgcaugaa 2700
   uacaucgaac uuaucgaaau ugcuaggaac ucgacucaag acagaauccu ugagaugaag 2760
   guaauggagu ucuuuaugaa gguuuaugga uaccgaggga agcaucucgg uggaucacga 2820
   aaacccgacg gagcaaucua uacggugggg agcccgauug auuacggagu gaucgucgac 2880
   acgaaagccu acagcggugg guacaaucuu cccaucgggc aggcagauga gaugcaacgu 2940
   uaugucgaag aaaaucagac caggaacaaa cacaucaauc caaaugagug guggaaagug 3000
   uauccuucau cagugaccga guuuaaguuu uuguuugucu cugggcauuu caaaggcaac 3060
   uauaaggccc agcucacacg guugaaucac auuacgaacu gcaauggugc gguuuugucc 3120
   guagaggaac ugcucauugg uggagaaaug aucaaagcgg gaacucugac acuggaagaa 3180
   gucagacgca aguuuaacaa uggcgagauc aauuuccgcu cauaaaaaau cagccucgac 3240
   ugugccuucu aguugccagc caucuguugu uugccccucc cccgugccuu ccuugacccu 3300
   ggaaggugcc acucccacug uccuuuccua auaaaaugag gaaauugcau cacaacacuc 3360
   aacccuaucu cggucuauuc uuuugauuua uaagggauuu ugccgauuuc ggccuauugg 3420
   uuaaaaaaug agcugauuua acaaaaauuu aacgcgaauu aauucugugg aauguguguc 3480
   aguuagggug uggaaagucc ccaggcuccc cagcaggcag aaguaugcaa agcaugcauc 3540
   ucaauuaguc agcaaccagg uguggaaagu ccccaggcuc cccagcaggc agaaguaugc 3600
   aaagcaugca ucucaauuag ucagcaacca uagucccgcc ccuaacuccg cccaucccgc 3660
   cccuaacucc gcccaguucc gcccauucuc cgccccaugg cugacuaauu uuuuuuauuu 3720
   augcagaggc cgaggccgcc ucugccucug agcuauucca gaaguaguga ggaggcuuuu 3780
   uuggaggc                                                          3788

## Beschreibung

Die Erfindung betrifft eine neue TAL-Effektornuklease (TALEN) zum gezielten Knockout des HIV-Korezeptors CCR5.

Neben seiner eigentlichen Funktion in der Zelle spielt der Chemokinrezeptor CCR5 eine wichtige Rolle bei der HIV-Infektion. Hier tritt er für die so genannten CCR5-tropen Stämme des HI-Virus als Korezeptor in Erscheinung, welcher die initiale HIV-Infektion vermittelt. Ist kein CCR5 auf der Oberfläche einer T-Helferzelle vorhanden, können die HI-Viren nicht mit der Wirtszelle verschmelzen, so dass es nicht zu einer Infektion kommt. So schützt eine homozygote Deletion (CCR5Δ32) im CCR5-Gen, die bei ca. 1% der Westeuropäer und der "weißen" Amerikaner ("Kaukasier") vorkommt, nahezu vollständig vor einer HIV-Infektion mit CCR-tropen Stämmen. Folglich ist CCR5 ein sehr interessantes Ziel für die HIV-Therapie.

Bisherige pharmakologische Ansätze, die auf eine Blockade des CCR5 abzielen, erfordern eine lebenslange Behandlung im Rahmen der kombinierten antiretroviralen Therapie ART. Dies ist langfristig mit potentiell schweren Nebenwirkungen, aber auch mit mangelnder Compliance der Patienten und Resistenzentwicklungen verbunden. Eine genetische Zerstörung ("Knockout") des CCR5 (im Sinne einer Gentherapie) wäre dagegen im Idealfall als Einmalbehandlung ausreichend, da der genetische Schutz an alle Tochterzellen weitergegeben wird. Dies wird nicht nur die durch die natürliche Resistenz CCR5Δ32-homozygoter Individuen belegt, sondern auch durch den Fallbericht der erfolgreichen Therapie einer HIV-Infektion bei dem so genannten "Berlin-Patienten" nach allogener Stammzelltransplantation mit CCR5Δ32-homozygoten Spenderzellen (Hütter G et al. Long-term control of HIV by CCRDelta32/Delta32 stem-cell transplantation. N Engl J Med. 2009, 360: 692-698; Allers K et al. Evidence for the cure of HIV infection by CCR5Δ32/Δ32 stem cell transplantation. Blood 2011; 117: 2791-2799).

Basierend auf diesen Beobachtungen wurden Konzepte zum genetischen Knockout des CCR5 bei HIV-Patienten entwickelt. Die derzeit erfolgversprechendsten Strategien basieren auf so genannten "Designernukleasen" (s. z.B. Manjunath N. et al., Newer Gene Editing Technologies toward HIV Gene Therapy, Viruses 2013, 5, 2748-2766). Diese Designernukleasen bestehen aus zwei Komponenten: einer Erkennungsdomäne, welche die Spezifität im Genom bestimmt und fast völlig frei designt werden kann, und einer Nukleasedomäne, welche an der gewählten Stelle im Genom einen Doppelstrangbruch induziert. Durch die fehlerhafte Reparatur dieses Doppelstrangbruches kommt es zu Verschiebungen des offenen Leserahmens des Zielgens und somit im Idealfall zu einem Knockout. Die ersten breit anwendbaren Designernukleasen waren die Zinkfingernukleasen (ZFN). Die Sangamo BioSciences, Inc., testet beispielsweise derzeit unter der Bezeichnung SB-728 eine von ihr entwickelte CCR5-spezifische Zinkfingernuklease (http://www.sangamo.com/pipeline/sb-728.html) für die klinische Anwendung (Tebas et al., Gene Editing of CCR5 in Autologous CD4 T Cells of Persons Infected with HIV. N Engl J Med 2014;370:901-10). Die klinische Studie zeigte die Machbarkeit des Ansatzes, jedoch konnte ein längerfristiger klinischer Nutzen auf die Viruslast nur bei einem Probanden beobachtet werden, der sich als heterozygot für die natürliche CCR5Δ32-Mutation erwies.

Bei den TAL-Effektornukleasen (transcription activator-like effector nucleases, TALEN) handelt es sich um die nächste Generation von Designernukleasen (s. z.B. Mussolino, C, Cathomen T. TALE nucleases: tailored genome engineering made easy, Curr Opin Biotechnol. 2012, 23(5): 644-50; WO 2011/072246 A2; EP 2510096 A2; WO 2011/154393 A1; WO 2011/159369 A1; WO 2012/093833 A2; WO 2013/182910 A2). Gegenüber ZFN zeichnen sie sich u.a. durch eine höhere Spezifität aus, so dass sich das Risiko von Off-target-Effekten, also dem Setzen von Mutationen an einer anderen als der gewünschten Stelle im Genom, deutlich verringert (Händel E-M, Cathomen T. Zinc-finger nuclease based genome surgery: it's all about specificity. Curr Gene Ther 2011, 11: 28-37; Mussolino C et al. A novel TALE nuclease scaffold enables high genome editing activity in combination with low toxicity. Nucleic Acids Res. 2011; 39: 9283-9293).

CCR5-spezifische TALEN sind bereits bekannt (s. z.B. Mussolino C et al. A novel TALE nuclease scaffold enables high genome editing activity in combination with low toxicity. Nucleic Acids Res. 2011; 39: 9283-9293; WO 2011/146121 A1; WO 2012/093833 A2; US 2013/0217131 A1, WO 2013/074999 A1), eine klinische Nutzung ist jedoch bisher nicht beschrieben. Sanjana et al. (Sanjana NE, Cong L, Zhou Y, Cunniff MM, Feng G, Zhang F, 2012, A transcription activator-like effector toolbox for genome engineering, Nature Protocols 7, 171-192 (2012), doi: 10.1038/nprot.2011.431) offenbaren ein Protokoll zur Konstruktion von TALEN. Fine et al. (Eli J. Fine, Thomas J. Cradick, Charles L. Zhao, Yanni Lin, Gang Bao, 2014, An online bioinformatics tool predicts zinc finger and TALE nuclease off-target cleavage, Nucleic Acids Research, 42, e42, doi: 10.1093/nar/gkt1326) beschreiben ein Programm zur Vorhersage von unspezifischer Aktivität von TALEN.

Es besteht nach wie vor ein Bedarf an Mitteln zur effizienten Behandlung einer HIV-Infektion. Aufgabe der Erfindung ist es daher, ein solches Mittel bereit zu stellen. Insbesondere ist es die Aufgabe der vorliegenen Erfindung, ein Mittel bereit zu stellen, mit dessen Hilfe ein effizienterer Knockout des HIV-Korezeptors CCR5 als bisher erreicht werden kann.

Gelöst wird die Aufgabe durch die Gegenstände des Anspruchs 1 und der weiteren nebengeordneten Ansprüche. Zweckmäßige Ausgestaltungen der erfindungsgemäßen Lösung sind in den Unteransprüchen angegeben.

In einem ersten Aspekt stellt die Erfindung ein TAL-Effektornuklease-Paar bereit, umfassend ein erstes und ein zweites TAL-Effektornuklease-Monomer, wobei jedes TAL-Effektornuklease-Monomer eine Endonukleasedomäne mit Typ-II-Endonukleaseaktivität und eine TAL-Effektor-DNA-Bindungsdomäne mit einer Mehrzahl von Wiederholungseinheiten, die jeweils ein variables Aminosäurepaar RVD aufweisen, umfasst, und wobei
a) die TAL-Effektor-DNA-Bindungsdomäne des ersten TAL-Effektornuklease-Monomers an die Zielsequenz GCTGGTCATCCTCATCCTG (SEQ ID NO: 1) bindet und die RVD-Sequenz NH HD NG NH NH NG HD NI NG HD HD NG HD NI NG HD HD NG NN umfasst,
   und
b) die TAL-Effektor-DNA-Bindungsdomäne des zweiten TAL-Effektornuklease-Monomers an die Zielsequenz AGATGTCAGTCATGCTCTT (SEQ ID NO: 2) bindet und die RVD-Sequenz NI NN NI NG NN NG HD NI NH NG HD NI NG NH HD NG HD NG NG umfasst.

Das erfindungsgemäße TAL-Effektornuklease-Paar ist in der Lage, mit bislang unerreicht hoher Effizienz von >50% einen Knockout des CCR5 in primären T-Lymphozyten zu bewirken. Dabei ermöglicht die Erfindung überraschend auch einen konsistenten biallelen Knockout beider CCR5-Allele und somit einen kompletten Schutz der modifizierten Zellen vor dem HIV-Eintritt, entgegen einer im Stand der Technik von führenden Experten geäußerten Ansicht, wonach dies derzeit nicht möglich sei ("Consistent nuclease-mediated biallelic knockdown is not yet tenable", s. Kay, M.A. und Walker, B.D., 2014, Engineering Cellular Resistance to HIV, N Engl J Med 370:968-969). Darüber hinaus hat sich herausgestellt, dass das erfindungsgemäße TALEN-Paar sich ausgezeichnet für einen auf mRNA-Transfektion basierenden (und damit besonders schonenden, sicheren und GMP-kompatiblen) Gentransfer eignet. Damit stellt die Erfindung erstmals ein auf einer Designernuklease basierendes Mittel für die HIV-Behandlung bereit, das eine hohe Knockout-Effizienz und -Selektivität mit geringen Off-Target-Effekten und sonstigen aus pharmakologischer Sicht vorteilhaften Eigenschaften verbindet.

Unter einer "TAL-Effektornuklease" oder "TALEN" (Transcription activator-like effector nuclease) wird hier ein Fusionsprotein verstanden, welches die DNA-Bindungsdomäne eines TAL-Effektors (TALE) und eine DNA-Spaltungsdomäne einer Restriktionsendonuklease enthält. Bei den TAL-Effektoren handelt es sich um DNA-bindende Proteine, die von Pflanzenpathogenen wie *Xanthomonas* spp. gebildet werden. Die DNA-Bindung der TAL-Effektoren wird über eine Domäne mit einer variierenden Anzahl (in der Regel 5 bis 30) von Wiederholungseinheiten ("repeats") aus meist 33 bis 35 Aminosäuren vermittelt. Jede Wiederholungseinheit verfügt dabei über zwei hochvariable Aminosäurereste (repeat-variable diresidue, RVD), in der Regel an den Positionen 12 und 13, die an genau eine Base einer DNA-Zielsequenz bindet. Die Beziehung zwischen RVD und DNA-Zielnukleotid ist im Folgenden wiedergegeben.

| RVD (Einbuchstabencode) | RVD (Dreibuchstabencode) | Nukleotid(e) |
|---|---|---|
| NH | Asn-His | G |
| HD | His-Asp | C |
| NG | Asn-Gly | T |
| NI | Asn-Ile | A |
| NN | Asn-Asn | R (G, A) |
| NK | Asn-Lys | G |
| NS | Asn-Ser | N (A, C, G, T) |

Unter einer "RVD-Sequenz" wird hier eine zusammenhängende Abfolge von RVDs in einer TAL-Effektor-Bindungsdomäne verstanden, wobei die Sequenz, sofern nichts anderes angegeben ist, hier in N-C-Richtung, d.h. vom N-terminus zum C-Terminus, angegeben ist. Dem Fachmann ist hier selbstverständlich bewusst, dass die RVDs einer "RVD-Sequenz" nicht unmittelbar in dem Sinne aufeinanderfolgen, dass sie selbst direkt kovalent miteinander verbunden sind, sondern dass die Wiederholungseinheiten ("repeats") direkt miteinander verbunden sind, in denen die RVDs enthalten sind, so dass die RVDs jeweils durch Aminosäuren der Grundstruktur der Wiederholungseinheiten getrennt sind.

Unter einer "Zielsequenz" oder "Target-Sequenz" wird hier eine Nukleotidsequenz, in der Regel eine DNA-Sequenz, verstanden, die von der TAL-Effektor-Bindungsdomäne gebunden wird.

Die hier offenbarten aus jeweils 19 RVDs bestehenden RVD-Sequenzen haben die folgenden Zielsequenzen (in 5'-3'-Richtung; Einbuchstabencode für die Aminosäuren):

Unter einer "Wiederholungseinheit" ("repeat") in Bezug auf eine TAL-Effektor-Bindungsdomäne wird hier eine zusammenhängende Abfolge von in der Regel 33-35, meist 34 Aminosäuren verstanden, die bis auf die hochvariablen RVDs an den Positionen 12 und 13 eine weitgehend identische Aminosäuresequenz aufweisen. Es ist möglich, dass auch innerhalb der konservierten Grundstruktur der Wiederholungseinheit, d.h. der im Wesentlichen gleichbleibenden Struktur, in die hochvariablen RVDs eingebettet sind, einzelne Aminosäuren variieren, beispielsweise an den Positionen 4, 10 und/oder 32 in einer Wiederholungseinheit aus 34 Aminosäuren. Eine typische Wiederholungseinheit kann beispielsweise die folgende Aminosäuresequenz haben (tiefgestellte Ziffern geben die Position innerhalb der Wiederholungseinheit an):
LTPX₄QVVAIX₁₀SX₁₂X₁₃GGKQALETVQRLLPVLCQX₃₂HG (SEQ ID NO: 5)

X steht für eine beliebige Aminosäure, wobei an den Positionen 12 und 13 die hypervariablen Aminosäuren der RVDs stehen. An Position 4 (X₄) können beispielsweise die Aminosäuren E, Q, D oder A stehen, an Position 32 (X₃₂) die Aminosäuren A oder D. An Position 10 kann beispielsweise A oder V stehen. Beispiele für Wiederholungseinheiten sind im Folgenden wiedergegeben (XX steht für die hypervariablen Aminosäuren der RVDs, variable Aminosäuren sind durch Unterstreichung kenntlich gemacht):
LTPEQVVAIASXXGGKQALETVQRLLPVLCQAHG (SEQ ID NO: 6)
LTPQQVVAIASXXGGKQALETVQRLLPVLCQAHG (SEQ ID NO: 7)
LTPDQVVAIASXXGGKQALETVQRLLPVLCQDHG (SEQ ID NO: 8)
LTPAQVVAIASXXGGKQALETVQRLLPVLCQDHG (SEQ ID NO: 9)
LTPEQVVAIVSXXGGKQALETVQRLLPVLCQAHG (SEQ ID NO: 10)

Die TAL-Effektor-Bindungsdomäne kann eine oder mehrere derartige Varianten von Wiederholungseinheiten beinhalten, wobei auch eine Mischung verschiedener Varianten in Frage kommt.

Die äußere, der Nukleasedomäne nächstgelegene Wiederholungseinheit kann weniger, beispielsweise nur die ersten 15, 16, 17, 18, 19 oder 20 Aminosäuren der übrigen Wiederholungseinheiten umfassen. Eine solche Wiederholungseinheit wird auch als "halbe Wiederholungseinheit" oder "half repeat" bezeichnet.

Unter einer "DNA-Bindungsdomäne" wird hier der Bereich eines Proteins verstanden, der die Bindung des Proteins an eine DNA herbeiführt. Im Falle der DNA-Bindungsdomäne eines TAL-Effektors geschieht dies mittels der oben näher beschriebenen Wiederholungseinheiten ("repeats").

Die Formulierung, wonach eine TAL-Effektor-DNA-Bindungsdomäne an eine Zielsequenz bindet, ist so zu verstehen, dass die TAL-Effektor-DNA-Bindungsdomäne auf Grund ihrer RVD-Sequenz spezifisch an die Zielsequenz bindet. Dabei ist es nicht erforderlich, wenn auch bevorzugt, dass jedem Nukleotid der Zielsequenz ein RVD in der Bindungsdomäne zugeordnet ist. Die Relation zwischen RVD-Sequenz der TAL-Effektor-DNA-Bindungsdomäne und Zielsequenz muss lediglich eine solche sein, dass eine spezifische Bindung an die Zielsequenz erfolgt. "Spezifisch" bedeutet in diesem Zusammenhang, dass die Bindung im Wesentlichen ausschließlich an die Zielsequenz erfolgt.

Unter einem "TAL-Effektornuklease-Monomer" wird eine TAL-Effektornuklease verstanden, die aus einer einzigen Polypeptidkette besteht. Unter einem "TAL-Effektornuklease-Paar" oder "TALEN-Paar" wird eine aus zwei TAL-Effektornuklease-Monomeren zusammengesetzte TALEN verstanden. Die Monomere repräsentieren einen linken oder rechten Arm einer TALEN, die an gegenüberliegende Stränge einer DNA binden und zusammen die Spaltung der DNA an einer Stelle bewirken.

Wenn hier unter Bezug auf ein TALEN-Paar von einer "linken" oder "rechten" TALEN oder einem "linken" und "rechten" TALEN-"Arm" gesprochen wird, reflektiert dies die Tatsache, dass in einem TALEN-Paar TALEN-Monomere paarweise eingesetzt werden, d.h. einen Strangbruch innerhalb einer doppelsträngigen DNA herbeiführen, indem ein Monomer eine Zielsequenz auf dem Sinnstrang bindet, während ein anderes TALEN-Monomer des TALEN-Paares eine Zielsequenz auf dem dazu komplementären Antisinn-Strang bindet, und zwar so dass die Nukleasedomänen zueinander gerichtet sind, in einem als "Spacer" bezeichneten gemeinsamen DNA-Bereich zwischen den Zielsequenzen zu liegen kommen und dort jeweils einen Einzelstrangbruch bewirken. "Linke" und "rechte" TALEN-Monomere sind somit die Teile eines solchen TALEN-Paares, wobei als "linke" TALEN häufig jene TALEN bezeichnet wird, die an den Sinnstrang bindet, während die "rechte" TALEN den komplementären Strang bindet. Wenn hier von einer "linken" oder "rechten" TALEN gesprochen wird, soll damit jedoch keine Festlegung verbunden sein, dass die "linke" TALEN den Sinnstrang und die "rechte" TALEN den dazu komplementären Strang bindet.

Die vorliegende Erfindung betrifft ein "TALEN-Paar", d.h. ein Paar aus zwei erfindungsgemäßen Monomeren, die jeweils einen linken oder rechten Arm einer TALEN repräsentieren.

Unter einer "Endonukleasedomäne mit Typ-II-Endonukleaseaktivität" wird ein Polypeptid verstanden, das die DNA-Spaltungsaktivität einer Restriktionsendonuklease aufweist und die DNA innerhalb oder in unmittelbarer Nähe der Erkennungssequenz schneidet, kein ATP benötigt und keine Methyltransferase-Aktivität besitzt. Unter einer "Endonukleasedomäne mit Typ-IIS-Endonukleaseaktivität" wird eine Domäne einer Typ-II-Endonuklease verstanden, deren Schnittstelle in unmittelbarer Nähe der Erkennungssequenz, jedoch nicht innerhalb derselben liegt.

Unter "CCR5" wird hier der menschliche CC-Motiv-Chemokin-Rezeptor 5 (auch als CD195, CMKBR5 oder CC-CKR5 bezeichnet) verstanden. Eine Sequenz des humanen CCR5 ist in SEQ ID NO: 11 wiedergegeben (s. NCBI-Zugriffsnummer NC_018914.2).

Unter einem "Vektor" wird hier ein Transportvehikel zur Übertragung einer meist fremden Nukleinsäure in eine lebende Empfängerzelle durch Transfektion oder Transduktion verstanden Unter einem "Gentransfer-Vektor" wird hier ein Vektor verstanden, mit dessen Hilfe ein Gen in eine Zelle eingebracht werden kann. (Gentransfer-)Vektoren sind dem Fachmann gut bekannt. Beispiele für Gentransfer-Vektoren sind Plasmide, virale Vektoren oder mRNA.

Unter einer "Nukleinsäure" wird ein Polymer verstanden, dessen Monomere Nukleotide sind. Ein Nukleotid ist eine Verbindung aus einem Zuckerrest, einer stickstoffhaltigen heterozyklischen organischen Base (Nukleotid- oder Nukleobase) und einer Phosphatgruppe. Der Zuckerrest ist in der Regel eine Pentose, im Falle von DNA Desoxyribose, im Falle von RNA Ribose. Die Verknüpfung der Nukleotide erfolgt über die Phosphatgruppe mittels einer Phosphodiesterbrücke in der Regel zwischen dem 3'-C-Atom der Zuckerkomponente eines Nukleosids (Verbindung aus Nukleobase und Zucker) und dem 5'-C-Atom der Zuckerkomponente des nächsten Nukleosids. Der Begriff "Nukleinsäure" umfasst beispielsweise DNA, RNA und DNA/RNA-Mischsequenzen. So wie er hier verwendet wird, betrifft der Begriff "Nukleinsäure" insbesondere eine isolierte Nukleinsäure. Unter einer "isolierten Nukleinsäure" wird eine aus ihrer natürlichen bzw. ursprünglichen Umgebung herausgelöste oder synthetisch hergestellte Nukleinsäure verstanden.

Der Begriff "umfassend" wird hier so verwendet, dass er sowohl einen Gegenstand definiert, der ausschließlich die unter den Begriff gefassten Merkmale aufweist, als auch einen Gegenstand, der über die unter den Begriff gefassten Merkmale hinaus auch noch weitere Merkmale aufweist. Die Definition eines Gegenstandes dadurch, dass er bestimmte Merkmale umfasst, schließt daher auch die Definition dieses Gegenstandes durch die abschließende Auflistung dieser Merkmale, d.h. durch das Vorhandensein ausschließlich dieser Merkmale ein.

In einer bevorzugten Ausführungsform des erfindungsgemäßen TAL-Effektornuklease-Paars liegt bei jedem der TAL-Effektornuklease-Monomere die Endonukleasedomäne in Bezug auf die TAL-Effektor-DNA-Bindungsdomäne C-terminal. Bevorzugt umfasst jede Wiederholungseinheit, mit Ausnahme der der Endonukleasedomäne am nächsten liegenden Wiederholungseinheit, 33 bis 35 Aminosäuren, vorzugsweise 34 Aminosäuren, wobei die RVDs sich in jeder Wiederholungseinheit an den Positionen 12 und 13 befinden. Besonders bevorzugt haben sämtliche Wiederholungseinheiten, außer dem "half repeat" die Aminosäuresequenz gemäß SEQ ID NO: 5, wobei an Position 4 E, Q, D oder A, an Position 10 A oder V und an Position 32 A oder D stehen kann. Die Grundstruktur der Wiederholungseinheiten kann für alle Wiederholungseinheiten gleich oder verschieden sein. Es können ein oder mehrere Wiederholungseinheiten an Positionen innerhalb der Grundstruktur, beispielsweise an den Positionen 4, 10 und/oder 32, hinsichtlich der Aminosäure variieren. Die der Endonukleasedomäne am nächsten liegende Wiederholungseinheit kann eine geringere Anzahl von Aminosäuren, beispielsweise 15, 16, 17, 18, 19 oder 20 Aminosäuren, umfassen, wobei die Aminosäuren in diesem Fall vorzugsweise den ersten 15, 16, 17, 18, 19 oder 20 Aminosäuren der übrigen Wiederholungseinheiten entsprechen. Dabei kann beispielsweise die Aminosäure an Position 4 abweichen, beispielsweise E, Q, D und A sein, und/oder die Aminosäure an Position 10 abweichen, beispielsweise V statt A sein.

Besonders bevorzugt ist die Endonukleasedomäne der TAL-Effektornuklease-Monomere eine Typ-IIS-Endonuklease-Domäne, besonders bevorzugt die DNA-Spaltungsdomäne der FokI-Endonuklease. Eine Aminosäuresequenz für eine geeignete FokI-Spaltungsdomäne ist in SEQ ID NO: 12 angegeben. Es kommen aber auch andere Typ-II-Endonuklease-Spaltungsdomänen in Frage. Typ-II-Endonukleasen sind dem Fachmann bekannt, und geeignete Spaltungsdomänen können mittels Routineuntersuchungen ermittelt werden.

In einer besonders bevorzugten Ausführungsform weist das erste TAL-Effektornuklease-Monomer eine Aminosäuresequenz gemäß SEQ ID NO: 3 und das zweite TAL-Effektornuklease-Monomer eine Aminosäuresequenz gemäß SEQ ID NO: 4 auf. In der SEQ ID NO: 3 ist dabei die linke TALEN (im Folgenden auch als CCR5-Uco-L oder linker Arm der CCR5-Uco bezeichnet) und in SEQ ID NO: 4 die rechte TALEN (im Folgenden auch als CCR5-Uco-R oder rechter Arm der CCR5-Uco bezeichnet) eines TALEN-Paares angegeben, die gemeinsam einen Doppelstrangbruch in der DNA-Sequenz des CCR5 innerhalb des zwischen den Zielsequenzen gemäß SEQ ID NO: 1 und SEQ ID NO: 2 liegenden Spacers bewirken. Die Reparatur dieses Doppelstrangbruch durch zelluläre Reparatursysteme (nonhomologes end-joining, NHEJ) führt mit hoher Wahrscheinlichkeit zu einer Leserahmenverschiebung und damit einem Knockout des CCR5.

In einem zweiten Aspekt betrifft die vorliegende Erfindung auch eine Nukleinsäure, kodierend für ein erfindungsgemäßes TAL-Effektornuklease-Paar nach dem ersten Aspekt. Die Nukleinsäure umfasst
a) eine erste Nukleinsäure, die für ein erstes TAL-Effektornuklease-Monomer kodiert, wobei das erste TAL-Effektornuklease-Monomer eine Endonukleasedomäne mit Typ-II-Endonukleaseaktivität und eine TAL-Effektor-DNA-Bindungsdomäne mit einer Mehrzahl von Wiederholungseinheiten umfasst, die jeweils ein variables Aminosäurepaar RVD aufweisen, und wobei die TAL-Effektor-DNA-Bindungsdomäne an die Zielsequenz GCTGGTCATCCTCATCCTG (SEQ ID NO: 1) bindet und die RVD-Sequenz NH HD NG NH NH NG HD NI NG HD HD NG HD NI NG HD HD NG NN umfasst, und
b) eine zweite Nukleinsäure, die für ein zweites TAL-Effektornuklease-Monomer kodiert, wobei das zweite TAL-Effektornuklease-Monomer eine Endonukleasedomäne mit Typ-II-Endonukleaseaktivität und eine TAL-Effektor-DNA-Bindungsdomäne mit einer Mehrzahl von Wiederholungseinheiten umfasst, die jeweils ein variables Aminosäurepaar RVD aufweisen, und wobei die TAL-Effektor-DNA-Bindungsdomäne an die Zielsequenz AGATGTCAGTCATGCTCTT (SEQ ID NO: 2) bindet und die RVD-Sequenz NI NN NI NG NN NG HD NI NH NG HD NI NG NH HD NG HD NG NG umfasst.

Die das erfindungsgemäße TALEN-Paar bildenden TALEN-Monomere sind bei diesem Aspekt der Erfindung zusammen in einer gemeinsamen Nukleinsäure kodiert. Es kann sich bei der Nukleinsäure beispielsweise um ein Plasmid oder einen anderen geeigneten (Gentransfer-)Vektor handeln. Geeignete Vektoren sowie Verfahren zu deren Herstellung und deren Verwendung sind im Stand der Technik gut gekannt. Gegebenenfalls kann die Nukleinsäure neben dem TALEN-Kode noch weitere Elemente enthalten, beispielsweise einen oder mehrerer Promotoren, sowie Polyadenylierungssignale etc.

Die das erfindungsgemäße TALEN-Paar bildenden TALEN-Monomere können auch getrennt in zwei Nukleinsäuren kodiert sein. In einem dritten Aspekt betrifft die vorliegende Erfindung daher auch eine Nukleinsäurezusammensetzung, umfassend
a) eine erste Nukleinsäure, die für das erste TAL-Effektornuklease-Monomer der erfindungsgemäßen TAL-Effektornuklease kodiert, und
b) eine zweite Nukleinsäure, die für das zweite TAL-Effektornuklease-Monomer der erfindungsgemäßen TAL-Effektornuklease kodiert.

Bevorzugt handelt es sich bei der ersten und zweiten Nukleinsäure jeweils um eine mRNA, besonders bevorzugt eine stabilisierte mRNA (s. z.B. Kallen K.-J. et al., A novel, disruptive vaccination technology, Hum Vaccin Immunother. Oct 1, 2013; 9(10): 2263-2276, doi: 10.4161/hv.25181; Kallen K.-J. und Theß A., A development that may evolve into a revolution in medicine: mRNA as the basis for novel, nucleotide-based vaccines and drugs, Ther Adv Vaccines. Jan 2014; 2(1): 10-31, doi: 10.1177/2051013613508729). Gegebenenfalls können die erste und zweite Nukleinsäure neben dem TALEN-Kode noch weitere Elemente enthalten, beispielsweise einen oder mehrerer Promotoren, sowie Polyadenylierungssignale etc. Geeignete mRNAs für den linken und rechten Arm einer erfindungsgemäßen TALEN sind beispielhaft in den SEQ ID NO: 17 und 18 angegeben.

Im Falle einer mRNA erfolgt deren Transport in die Zielzelle(n), z.B. T-Lymphozyten, besonders bevorzugt mittels des von Berdien et al. (Berdien B et al., TALEN-mediated editing of endogenous T-cell receptors facilitates efficient reprogramming of T lymphocytes by lentiviral gene transfer, Gene Therapy, 2014, doi:10.1038/gt.2014.26) beschriebenen Verfahrens. Besonders bevorzugt werden beide TALEN-Arme (rechter und linker Arm) gleichzeitig in eine Zelle gebracht.

Die Einbringung des erfindungsgemäßen TALEN-Paares über mRNA hat für die klinische Anwendung eine Reihe entscheidender Vorteile. Der Einsatz eines DNA-basierten Gentransfervektors kann vermieden werden, was die Herstellung und praktische Anwendung entschieden vereinfacht. Die mRNA-vermittelte Expression der TALEN erfolgt nur vergleichsweise kurz, da die mRNA in der Zielzelle rasch abgebaut wird. Dadurch wird das Risiko von Off-target-Effekten weiter verringert. Darüber hinaus müssen die Zielzellen nur für sehr kurze Zeit in vitro kultiviert werden. Die entsprechende Technologie ist leicht an GMP-Erfordernisse anzupassen. Es sind, im Gegensatz zu viralen oder Plasmidvektoren, keine Nebenwirkungen durch den Gentransfer selbst (z.B. Insertionsmutagenese) oder durch eine langandauernde TALEN-Expression (Off-target-Effekte, Aktivierung einer TALENspezifischen Immunantwort) infolge unerwünschter Vektorinsertion zu erwarten.

In weiteren Aspekten betrifft die vorliegende Erfindung auch einen Vektor, insbesondere Gentransfer-Vektor, umfassend eine erfindungsgemäße Nukleinsäure, und eine isolierte Wirtszelle, umfassend einen erfindungsgemäßen Vektor, eine erfindungsgemäße Nukleinsäure oder Nukleinsäurezusammensetzung, wobei es sich bei der isolierten Wirtszelle um keine Keimbahnzelle eines menschlichen Lebewesens, insbesondere keine menschliche Keimzelle oder menschliche embryonale Keimzelle handelt, und wobei es sich um keine menschliche embryonale Stammzelle handelt, bei deren Gewinnung ein menschlicher Embryo zerstört worden ist oder wird.

In noch einem weiteren Aspekt betrifft die vorliegende Erfindung eine pharmazeutische Zusammensetzung, umfassend eine Nukleinsäure, Nukleinsäurezusammensetzung oder einen Vektor gemäß der vorliegenden Erfindung. Die pharmazeutische Zusammensetzung kann Adjuvantien umfassen, beispielsweise Lösungsmittel, Lösungsvermittler, Lösungsbeschleuniger, Salzbildner, Salze, Puffersubstanzen, Viskositäts- und Konsistenzbeeinflusser, Gelbildner, Emulgatoren, Solubilisatoren, Benetzer, Spreizmittel, Antioxidantien, Konservierungsmittel, Füll- und Trägerstoffe, etc.

In noch einem weiteren Aspekt betrifft die vorliegende Erfindung ein Arzneimittel, umfassend eine Nukleinsäure, Nukleinsäurezusammensetzung, einen Vektor oder eine pharmazeutische Zusammensetzung gemäß der vorliegenden Erfindung.

Die Erfindung wird im Folgenden anhand von Ausführungsbeispielen und der angehängten Figuren zu Veranschaulichungszwecken näher erläutert.

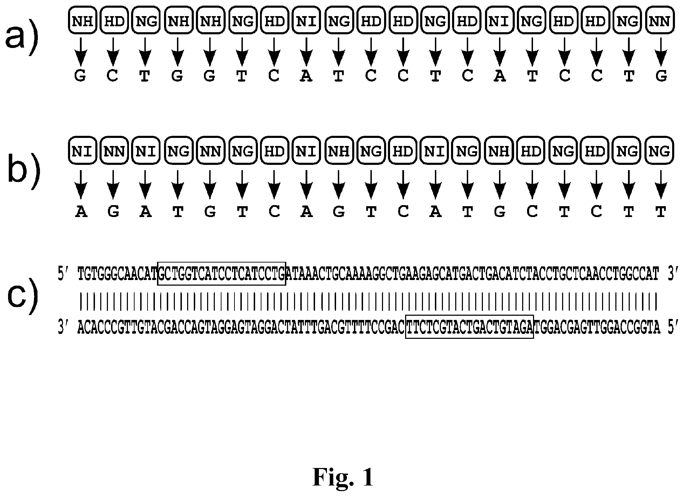

Fig. 1 Schematische Darstellung der DNA-Bindungsdomänen eines erfindungsgemäßen CCR5-spezifischen TALEN-Paares ("CCR5-Uco") und ihrer Zielsequenzen im CCR5-Gen. Die jeweils untere Zeile zeigt die Zielsequenzen für a) den linken und b) den rechten TALEN-Arm, die jeweils obere Zeile die relevanten RVDs (repeat variable di-residues) der entsprechenden TALE-Monomere in den Kästchen (Aminosäuren sind im Ein-Buchstaben-Code angegeben); c) Abschnitt (nt 135-221 der Sequenz gemäß SEQ ID NO: 11) der CCR5-DNA mit komplementärem Strang. Die Zielsequenzen des linken (oben) und rechten (unten, auf komplementärem Strang) Armes der CCR5-Uco-TALEN sind durch Umrahmung hervorgehoben.

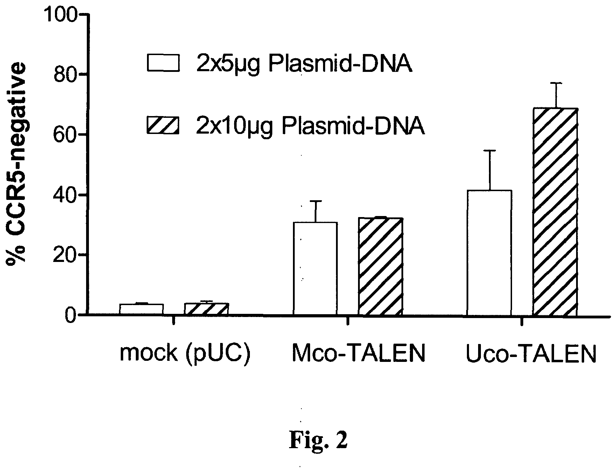

Fig. 2 Effizienzvergleich zwischen erfindungsgemäßer CCR5-TALEN ("Uco") und einer Kontroll-CCR5-TALEN ("Mco") aus dem Stand der Technik. Die Testung erfolgte durch Plasmidtransfektion in eine CCR5-positive, 293T-Zell-basierte Reporterzelllinie. Für alle getesteten Konstrukte wurden vergleichbare Transfektionseffizienzen beobachtet (anhand der Kotransfektion von eGFP). Der CCR5-Knockout wurde 6 Tage nach der Transfektion des CCR5+ 293T-Zellklons mit Hilfe eines spezifischen Antikörpers (anti-CD 195-APC-Cy-7 antibody) bestimmt (n=2). Für die "Mock"-Kontrolle wurden die Zellen mit einem irrelevanten Kontrollplasmid (pUC), welches kein TALEN kodiert, transfiziert.

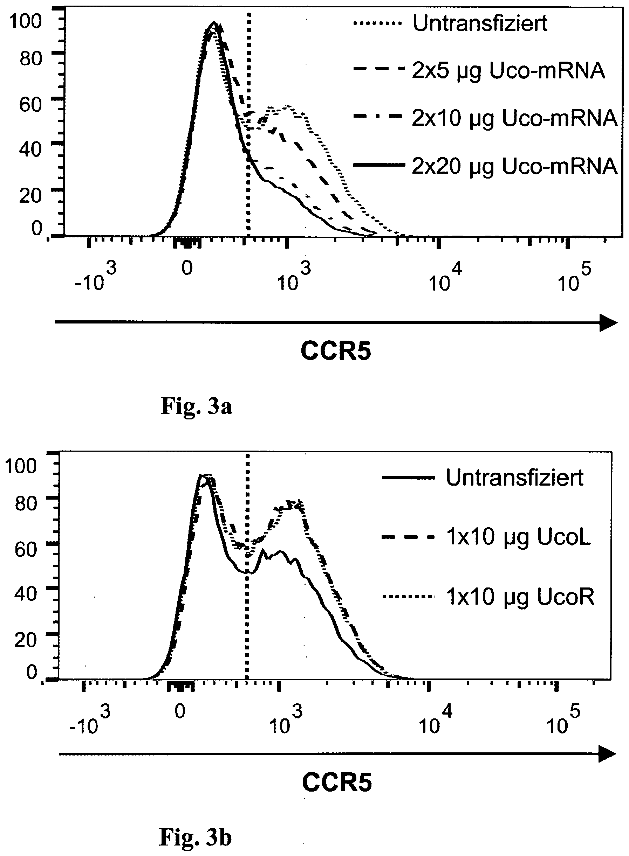

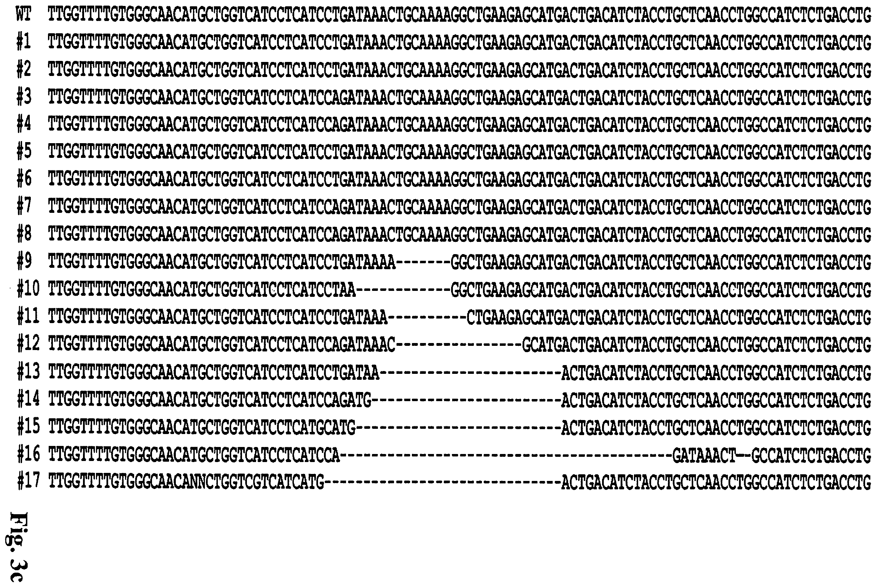

Fig. 3: CCR5-Knockout in primären T-Lymphozyten mit CCR5-Uco nach mRNA-Transfektion. Nach Ex-vivo-Aktivierung exprimieren ca. die Hälfte der primären T-Lymphozyten eines gesunden Probanden CCR5 (= Zellen rechts der gestrichelten Linie, Vgl. "untransfiziert"). a) Nach Transfektion der CCR5-spezifischen TALEN (Uco) nimmt der Anteil CCR5-positiver Zellen mit wachsender Menge transfizierter mRNA (umgekehrt proportional) ab. b) Die Kontrolltransfektion der einzelnen TALEN-Arme führt dagegen nicht zu einer Abnahme des Anteils CCR5-positiver Zellen. Der CCR5-Knockout wurde 6 Tage nach der mRNA-Transfektion mit Hilfe eines spezifischen Antikörpers (anti-CD 195-PerCP-Cy5.5 antibody) bestimmt. c) Eine Analyse der Zielstelle der CCR5-Uco-TALEN zeigt einen genetischen Knockout in 9 von 17 (>50%) analysierten primären T-Lymphozyten

### Beispiele

Es wurde eine erfindungsgemäße CCR5-spezifische TALEN (im Folgenden "CCR5-Uco") hergestellt und untersucht. Die erfindungsgemäße TALEN unterscheidet sich in der von ihr erkannten Zielsequenz im CCR5-Gen (s. Fig. 1) von den bisher beschriebenen TALEN.

Gegenüber einer kodonoptimierten CCR5-TALEN ("Mco"; s. SEQ ID NO: 13, 14), die auf publizierten Arbeiten des Labors von Prof. Toni Cathomen (Freiburg) basiert (s. Mussolino C et al., A novel TALE nuclease scaffold enables high genome editing activity in combination with low toxicity, Nucleic Acids Res. 2011, 39: 9283-9293), zeigt die erfindungsgemäße CCR5-Uco-TALEN eine deutlich höhere Rate der Induktion des CCR5-Knockouts nach Plasmidtransfektion in eine Reporterzelllinie (s. Fig. 2). Die Nukleinsäuresequenz der für den Einsatz in humanen Zellen kodonoptimierten TALEN-Bausteine basiert dabei auf den Veröffentlichungen der Gruppe um Feng Zhang (Zhang et al., Efficient construction of sequence-specific TAL effectors for modulating mammalian transcription, Nature Biotechnology, 2011, 29, 149-153; Sanjana NE et al., A TAL Effector Toolbox for Genome Engineering, Nature Protocols, 2012, 7: 171-192).

Die RVD-Sequenzen der CCR5-Mco-TALEN gemäß dem Stand der Technik sind wie folgt: Linker Arm (L) = NN NG NN NN NN HD NINI HD NI NG NN HD NG NN NN NG HD; Rechter Arm (R) = HD NG NG HD NI NN HD HD NG NG NG NG NN HD NI NN NG NG. Die dazugehörige Erkennungssequenz auf DNA-Ebene:
L auf Sinnstrang = GTGGGCAACATGCTGGTC (SEQ ID NO: 15);
R auf Antisinnstrang = CTTCAGCCTTTTGCAGTT (SEQ ID NO: 16).
Die Länge des Spacers beträgt 15 nt. Auch hier basiert die Herstellung der TALEN-Plasmide auf den Veröffentlichungen von Zhang F, bzw. Sanjana NE et al. (siehe oben).

Mittels mRNA-Transfektion (s. Berdien B et al., TALEN-mediated editing of endogenous T-cell receptors facilitates efficient reprogramming of T lymphocytes by lentiviral gene transfer, Gene Therapy, 2014, doi:10.1038/gt.2014.26) mit der erfindungsgemäßen CCR5-Uco wurde in primären T-Lymphozyten ein CCR5-Knockout bewirkt (s. Fig. 3). Nukleinsäuresequenzen der hierfür verwendeten mRNA sind in den SEQ ID NO: 17 und 18 wiedergegeben. In SEQ ID NO: 17 ist die mRNA des linken TALEN-Arms, in SEQ ID NO: 18 die mRNA des rechten TALEN-Arms wiedergegeben. Die Nukleotide 10-3225 der mRNAs in SEQ ID NO: 17 und 18 kodieren jeweils die TALEN-Arme (Monomere), deren Aminosäuresequenzen in den SEQ ID NO: 3, 4 angegeben sind.

Die transfizierte mRNA wurde über den T7-Promoter nach AvrII-Linearisierung des Vektors hergestellt. Da die AvrII-Schnittstelle 563bp hinter dem Stopcodon liegt, ist die angegebene Sequenz länger als der offene Leserahmen. Nach Linearisierung wurde die jeweilige Uco-TALEN-DNA als Vorlage für die Herstellung der mRNA mittels T7 mScript™ Standard mRNA Production System von Cellscript (Madison, WI 53713 USA) verwendet. Nach Herstellerangaben wurde die mRNA mit einem 5'Cap- und einem Poly-A-Schwanz versehen. Die Transfektion der mRNA erfolgte über die Elektroporation der primären T-Zellen für 10 ms bei 300V. Mit der Mco-CCR5-TALEN gelang es dagegen nicht, einen CCR5-Knockout in primären T-Zellen bzw. Z-Zelllinien zu realisieren (während ein k.o. des T-Zellrezeptors möglich war, s. Berdien et al 2014, s. oben). Erst mit der erfindungsgemäßen CCR5-Uco-TALEN gelang es, mittels mRNA-Transfer einen signifikanten Anteil von >50% der CCR5-Allele auszuschalten (Abb. 3c). Dies legt nahe, dass nur ausreichend aktive TALEN in der Lage sind, nach einer mRNA-Transfektion ihre Funktion in primären T-Zellen zu erfüllen. Somit ist die erfindungsgemäße CCR5-TALEN für einen Einsatz per mRNA-Transfektion besonders gut geeignet, was sie für eine klinische Anwendung äußerst attraktiv macht.

**Sequenzübersicht:**

| SEQ ID NO: | Typ | Beschreibung |
|---|---|---|
| 01 | DNA | Zielsequenz TALEN CCR5-Uco L |
| 02 | DNA | Zielsequenz TALEN CCR5-Uco R |
| 03 | PRT | TALEN CCR5-Uco L |
| 04 | PRT | TALEN CCR5-Uco R |
| 05 | PRT | Wiederholungssequenz (Konsensus) |
| 06 | PRT | Wiederholungssequenz |
| 07 | PRT | Wiederholungssequenz |
| 08 | PRT | Wiederholungssequenz |
| 09 | PRT | Wiederholungssequenz |
| 10 | PRT | Wiederholungssequenz |
| 11 | DNA | hCCR5 |
| 12 | PRT | FokI-Spaltungsdomäne |
| 13 | PRT | TALEN CCR5-Mco L |
| 14 | PRT | TALEN CCR5-Mco R |
| 15 | DNA | Zielsequenz TALEN CCR5-Mco L |
| 16 | DNA | Zielsequenz TALEN CCR5-Mco R |
| 17 | mRNA | mRNA CCR5-Uco L |
| 18 | mRNA | mRNA CCR5-Uco R |

### SEQUENZPROTOKOLL - FREIER TEXT

TALEN repeat = TALEN-Wiederholungseinheit
Any amino acid, or E, Q, D or A = Beliebige Aminosäure, oder E, Q, D oder A
Any amino acid, or A or V = Beliebige Aminosäure, oder A oder V
Any amino acid, or A or D = Beliebige Aminosäure, oder A oder D
Repeat variable diresidue (RVD) = "Repeat variable diresidue (RVD; variabler Zweichfachrest der Wiederholungseinheit)
FokI cleavage domain = FokI-Spaltungsdomäne

## Patentansprüche

1. TAL-Effektornuklease-Paar, umfassend ein erstes und ein zweites TAL-Effektornuklease-Monomer, wobei jedes TAL-Effektornuklease-Monomer eine Endonukleasedomäne mit Typ-II-Endonukleaseaktivität und eine TAL-Effektor-DNA-Bindungsdomäne mit einer Mehrzahl von Wiederholungseinheiten, die jeweils ein variables Aminosäurepaar RVD aufweisen, umfasst, und wobei
a) die TAL-Effektor-DNA-Bindungsdomäne des ersten TAL-Effektornuklease-Monomers an die Zielsequenz GCTGGTCATCCTCATCCTG (SEQ ID NO: 1) bindet und die RVD-Sequenz NH HD NG NH NH NG HD NI NG HD HD NG HD NI NG HD HD NG NN umfasst,
und
b) die TAL-Effektor-DNA-Bindungsdomäne des zweiten TAL-Effektornuklease-Monomers an die Zielsequenz AGATGTCAGTCATGCTCTT (SEQ ID NO: 2) bindet und die RVD-Sequenz NI NN NI NG NN NG HD NI NH NG HD NI NG NH HD NG HD NG NG umfasst.

2. TAL-Effektornuklease-Paar nach Anspruch 1, wobei bei jedem der TAL-Effektornuklease-Monomere die Endonukleasedomäne in Bezug auf die TAL-Effektor-DNA-Bindungsdomäne C-terminal liegt und jede Wiederholungseinheit mit Ausnahme der der Endonukleasedomäne am nächsten liegenden 33 bis 35 Aminosäuren, vorzugsweise 34 Aminosäuren, umfasst, wobei die RVDs sich in jeder Wiederholungseinheit an den Positionen 12 und 13 befinden.

3. TAL-Effektornuklease-Paar nach Anspruch 1 oder 2, wobei die Endonukleasedomäne der TAL-Effektornuklease-Monomere eine DNA-Spaltungsdomäne der FokI-Endonuklease ist.

4. TAL-Effektornuklease-Paar nach einem der vorhergehenden Ansprüche, wobei das erste TAL-Effektornuklease-Monomer eine Aminosäuresequenz gemäß SEQ ID NO: 3 und das zweite TAL-Effektornuklease-Monomer eine Aminosäuresequenz gemäß SEQ ID NO: 4 aufweist.

5. Nukleinsäure, kodierend für ein TAL-Effektornuklease-Paar nach Anspruch 1.

6. Vektor, umfassend eine Nukleinsäure nach Anspruch 5.

7. Nukleinsäurezusammensetzung, umfassend
a) eine erste Nukleinsäure, die für das erste TAL-Effektornuklease-Monomer nach Anspruch 1 kodiert, und
b) eine zweite Nukleinsäure, die für das zweite TAL-Effektornuklease-Monomer nach Anspruch 1 kodiert.

8. Nukleinsäurezusammensetzung nach Anspruch 7, wobei die erste Nukleinsäure eine erste mRNA und die zweite Nukleinsäure eine zweite mRNA ist.

9. Nukleinsäurezusammensetzung nach Anspruch 8, wobei die erste Nukleinsäure eine Sequenz gemäß SEQ ID NO: 17 und die zweite Nukleinsäure eine Sequenz gemäß SEQ ID NO: 18 umfasst.

10. Isolierte Wirtszelle, umfassend eine Nukleinsäure nach Anspruch 5, eine Nukleinsäurezusammensetzung nach einem der Ansprüche 7 bis 9 oder einen Vektor nach Anspruch 6, mit der Maßgabe, dass die Wirtszelle keine menschliche Keimzelle oder menschliche embryonale Keimzelle ist, und keine menschliche embryonale Stammzelle ist, bei deren Gewinnung ein menschlicher Embryo zerstört worden ist oder wird.

11. Pharmazeutische Zusammensetzung, umfassend eine Nukleinsäure nach Anspruch 5, eine Nukleinsäurezusammensetzung nach einem der Ansprüche 7 bis 9, oder einen Vektor nach Anspruch 6.

12. Arzneimittel, umfassend eine Nukleinsäure nach Anspruch 5, eine Nukleinsäurezusammensetzung nach einem der Ansprüche 7 bis 9, einen Vektor nach Anspruch 6 oder eine pharmazeutische Zusammensetzung nach Anspruch 11.

## Claims

1. A TAL effector nuclease pair, comprising a first and a second TAL effector nuclease monomer, wherein each TAL effector nuclease monomer comprises an endonuclease domain with Type II endonuclease activity and a TAL effector DNA-binding domain with a plurality of repeats, each having a variable amino acid pair RVD, and wherein
a) the TAL effector DNA-binding domain of the first TAL effector nuclease monomer binds to the target sequence GCTGGTCATCCTCATCCTG (SEQ ID NO: 1) and comprises the RVD sequence NH HD NG NH NH NG HD NI NG HD HD NG HD NI NG HD HD NG NN,
and
b) the TAL effector DNA-binding domain of the second TAL effector nuclease monomer binds to the target sequence AGATGTCAGTCATGCTCTT (SEQ ID NO: 2) and comprises the RVD sequence NI NN NI NG NN NG HD NI NH NG HD NI NG NH HD NG HD NG NG.

2. The TAL effector nuclease pair as claimed in claim 1, wherein the endonuclease domain in each of the TAL effector nuclease monomers is C-terminal with respect to the TAL effector DNA-binding domain and each repeat, with the exception of that which lies immediately adjacent to the endonuclease domain comprises 33 to 35 amino acids, preferably 34 amino acids, wherein the RVDs are in positions 12 and 13 in each repeat.

3. The TAL effector nuclease pair as claimed in claim 1 or claim 2, wherein the endonuclease domain of the TAL effector nuclease monomers is a DNA cleavage domain of FokI endonuclease.

4. The TAL effector nuclease pair as claimed in one of the preceding claims, wherein the first TAL effector nuclease monomer comprises an amino acid sequence in accordance with SEQ ID NO: 3 and the second TAL effector nuclease monomer comprises an amino acid sequence in accordance with SEQ ID NO: 4.

5. A nucleic acid coding for a TAL effector nuclease pair as claimed in claim 1.

6. A vector comprising a nucleic acid as claimed in claim 5.

7. A nucleic acid composition comprising:
a) a first nucleic acid which codes for the first TAL effector nuclease monomer as claimed in claim 1, and
b) a second nucleic acid which codes for the second TAL effector nuclease monomer as claimed in claim 1.

8. A nucleic acid composition as claimed in claim 7, wherein the first nucleic acid is a first mRNA and the second nucleic acid is a second mRNA.

9. The nucleic acid composition as claimed in claim 8, wherein the first nucleic acid comprises a sequence in accordance with SEQ ID NO: 17 and the second nucleic acid comprises a sequence in accordance with SEQ ID NO: 18.

10. An isolated host cell comprising a nucleic acid as claimed in claim 5, a nucleic acid composition as claimed in one of claims 7 to 9 or a vector as claimed in claim 6, with the proviso that the host cell is not a human gamete or human embryonic gamete, and is not a human embryonic stem cell which has been obtained or is obtained by destroying a human embryo.

11. A pharmaceutical composition comprising a nucleic acid as claimed in claim 5, a nucleic acid composition as claimed in one of claims 7 to 9, or a vector as claimed in claim 6.

12. A medicament comprising a nucleic acid as claimed in claim 5, a nucleic acid composition as claimed in one of claims 7 to 9, a vector as claimed in claim 6 or a pharmaceutical composition as claimed in claim 11.

## Revendications

1. Paire de nucléases effectrices TAL, comprenant un premier et un deuxième monomères de nucléase effectrice TAL, chacun des monomères de nucléase effectrice TAL comprenant un domaine d'endonucléase ayant une activité d'endonucléase de type II et un domaine de liaison effecteur TAL - ADN comportant une pluralité d'unités répétitives dont chacune comporte une paire d'acides aminés variable RVD, et
a) le domaine de liaison effecteur TAL - ADN du premier monomère de nucléase effectrice TAL entrant en liaison avec la séquence cible GCTGGTCATCCTCATCCTG (SEQ ID N° : 1), et la séquence RVD comprenant NH HD NG NH NH NG HD NI NG HD HD NG HD NI NG HD HD NG NN,
et
b) le domaine de liaison effecteur TAL - ADN du deuxième monomère de nucléase effectrice TAL entrant en liaison avec la séquence cible AGATGTCAGTCATGCTCTT (SEQ ID N° : 2) et la séquence RVD comprenant NI NN NI NG NN NG HD NI NH NG HD NI NG NH HD NG HD NG NG.

2. Paire de nucléases effectrices TAL selon la revendication 1, le domaine d'endonucléase se trouvant, chez chacun des monomères d'endonucléase effectrice, en situation C-terminale par rapport au domaine de liaison effecteur TAL - ADN, et chacune des unités répétitives, à l'exception de celle qui est la plus proche du domaine d'endonucléase, comprenant 33 à 35 acides aminés, de préférence 34 acides aminés, les RVDs se trouvant aux positions 12 et 13 dans chacune des unités répétitives.

3. Paire de nucléases effectrices TAL selon les revendications 1 ou 2, le domaine d'endonucléase des monomères de nucléase effectrice TAL étant un domaine de clivage d'ADN de l'endonucléase FokI.

4. Paire de nucléases effectrices TAL selon l'une des revendications précédentes, le premier monomère de nucléase effectrice TAL comportant une séquence d'acides aminés selon SEQ ID N° : 3 et le deuxième monomère de nucléase effectrice TAL comportant une séquence d'acides aminés selon SEQ ID N° : 4.

5. Acide nucléique codant pour une paire de nucléases effectrices TAL selon la revendication 1.

6. Vecteur, comprenant un acide nucléique selon la revendication 5.

7. Composition d'acides nucléiques, comprenant
a) un premier acide nucléique codant pour le premier monomère de nucléase effectrice TAL selon la revendication 1, et
b) un deuxième acide nucléique codant pour le deuxième monomère de nucléase effectrice TAL selon la revendication 1.

8. Composition d'acides nucléiques selon la revendication 7, le premier acide nucléique étant un premier ARNm et le deuxième acide nucléique étant un deuxième ARNm.

9. Composition d'acides nucléiques selon la revendication 8, le premier acide nucléique comprenant une séquence selon SEQ ID N° : 17 et le deuxième acide nucléique comprenant une séquence selon SEQ ID N° : 18.

10. Cellule hôte isolée, comprenant un acide nucléique selon la revendication 5, une composition d'acides nucléiques selon l'une des revendications 7 à 9, ou un vecteur selon la revendication 6, à condition que ladite cellule hôte ne soit ni un gamète humain, ni un gamète d'embryons humains, ni une cellule souche d'embryons humains laquelle est ou a été obtenue en détruisant un embryon humain.

11. Composition pharmaceutique comprenant un acide nucléique selon la revendication 5, une composition d'acides nucléiques selon l'une des revendications 7 à 9, ou un vecteur selon la revendication 6.

12. Médicament comprenant un acide nucléique selon la revendication 5, une composition d'acides nucléiques selon l'une des revendications 7 à 9, un vecteur selon la revendication 6 ou une composition pharmaceutique selon la revendication 11.
